# FASCICULE DE BREVET EUROPEEN

(11) **EP 3 994 122 B1**
(45) Date de publication et mention de la délivrance du brevet: **04.09.2024**
(21) Numéro de dépôt: 20750312.9
(22) Date de dépôt: 06.07.2020
(51) Int. Cl.: C07C 259/06, A61K 31/165, A61P 25/04, A61P 25/22, A61P 25/24

(54) **N-FORMYLHYDROXYLAMINES EN TANT QU'INHIBITEURS DE LA NEPRILYSINE (NEP), EN PARTICULIER EN TANT QU'INHIBITEURS MIXTES DE L'AMINOPEPTIDASE N (APN) ET DE LA NEPRILYSINE (NEP)**
N-FORMYLHYDROXYLAMINE ALS NEPRILYSIN (NEP)-INHIBITOREN, INSBESONDERE ALS MISCHINHIBITOREN VON AMINOPEPTIDASE N (APN) UND NEPRILYSIN (NEP)
N-FORMYLHYDROXYLAMINES AS NEPRILYSIN (NEP) INHIBITORS, IN PARTICULAR AS MIXED INHIBITORS OF AMINOPEPTIDASE N (APN) AND NEPRILYSIN (NEP)

(30) Priorité: 05.07.2019 FR 1907537
(43) Date de publication de la demande: 11.05.2022
(73) Titulaire: KOS THERAPEUTICS, INC., Raleigh, NC 276090 (US)
(72) Inventeur: PORAS, Hervé, 78450 VILLEPREUX (FR); FOURNIE-ZALUSKI, Marie-Claude, 75011 PARIS (FR); ROQUES, Bernard, 75014 PARIS (FR)
(74) Mandataire: Regimbeau
(86) Numéro de dépôt international: PCT/FR2020/051190
(87) Numéro de publication internationale: WO 2021/005294

(56) Documents cités:
- WO-A1-00/43001
- FR-A1- 2 518 088
- F. NOBLE, ET AL.: "Protection of endogenous enkephalin catabolism as natural approach to novel analgesic and antidepressant drugs", EXPERT OPINION ON THERAPEUTIC TARGETS, vol. 11, no. 2, 17 January 2007 (2007-01-17), Informa Healthcare, London, GB, pages 145 - 159, XP009171198, ISSN: 1472-8222, DOI: 10.1517/14728222.11.2.145
- M.C. FOURNIÉ-ZALUSKI, ET AL.: "New bidentates as full inhibitors of enkephalin-degrading enzymes: synthesis and analgesic properties", JOURNAL OF MEDICINAL CHEMISTRY, vol. 28, no. 9, 1 September 1985 (1985-09-01), American Chemical Society, Washington, DC, US, pages 1158 - 1169, XP002019770, ISSN: 0022-2623, DOI: 10.1021/jm00147a007

## Description

### DOMAINE DE L'INVENTION

L'invention porte sur de nouveaux dérivés N-Formylhydroxylamines et leur utilisation en tant qu'inhibiteurs mixtes de la néprilysine (NEP), et avantageusement en tant qu'inhibiteurs mixtes de la NEP et de l'aminopeptidase N (APN), des enzymes impliquées dans la dégradation des enképhalines, notamment dans le traitement de la douleur.

### ARRIERE PLAN DE L'INVENTION

Au cours de ces dernières années, la recherche en sciences de la vie a considérablement attiré les scientifiques à étudier les métalloenzymes et leurs modulateurs d'activité (inhibiteurs et / ou activateurs) afin d'améliorer la santé humaine en sélectionnant ainsi de nouvelles cibles thérapeutiques.

La plus grande catégorie de métalloprotéines est constituée d'enzymes de zinc. Au cours des dernières années, des preuves substantielles ont été accumulées impliquant les enzymes à Zn²⁺ dans la physiopathologie et la pathogenèse d'une grande variété de désordres humains allant des infections au cancer.

Les métalloprotéases à Zn²⁺ représentent un groupe important d'hydrolases impliquées dans de nombreux processus de régulation physiologique telle que la respiration, la pression artérielle, le transit intestinal, la sensation douloureuse ou la sensation de bien-être, le maintien de la réorganisation de la matrice extracellulaire au niveau des articulations, l'angiogenèse, l'homéostasie des protéines essentielles à l'activité cérébrale, le contrôle des équilibres hydriques et caloriques etc.... Ces enzymes sont responsables, soit de la maturation (formation d'une molécule active à partir d'un précurseur inactif), soit de l'inactivation (formation de métabolites inactifs à partir d'une molécule active) de peptides ou de protéines. Ainsi, on peut citer les enképhalines, peptides impliqués dans le contrôle de la douleur, qui sont dégradées en peptides inactifs par deux métalloprotéases à zinc, l'amino peptidase neutre (APN) (Meek J.L. et al. (1977), Neuropharmacology, 16, 151-154*)* et la néprilysine (NEP) (Malfroy B. et al. (1978), Nature, 276, 523-526)*.* La caractéristique générale des métalloprotéases à zinc est la présence d'au moins un cation Zn²⁺, indispensable à l'activité d'hydrolase de l'enzyme. En conséquence la stratégie classique de développement d'inhibiteurs efficaces de ce type d'enzymes consiste à concevoir une molécule reconnaissant les différents sous-sites de liaison de l'enzyme et possédant un groupement ayant une forte affinité pour le Zn²⁺ (revue Roques B.P. et al. (1993), PharmacolRev, 45, 87-146 ; Roques B.P. et al. (2000), TIPS, 21, 475-483)*.*

L'ion Zn²⁺ est essentiel pour l'activité catalytique de ces enzymes et est situé au niveau du site actif de l'enzyme, participant directement au mécanisme catalytique par le biais d'une interaction avec la molécule de substrat subissant la transformation. Le changement de coordination du Zn²⁺ est semble-t-il responsable d'un changement de conformation de la protéine qui induit une inhibition de l'activité de l'enzyme. Compte tenu de ce mécanisme, la structure classique d'un inhibiteur de ces métalloenzymes à zinc, contient un groupement qui est un bon chélatant du Zn²⁺ et qui est capable de se comporter comme un monodentate ou comme un bidentate vis à vis de Zn²⁺, afin de conduire à des complexes enzyme-inhibiteur dans lesquels le Zn²⁺ sera tétracoordiné ou pentacoordiné.

Ainsi, des composés comportant des groupements chélatant du Zn²⁺ peuvent être envisagés en tant qu'inhibiteurs des enzymes NEP, et même avantageusement en tant qu'inhibiteurs mixtes des enzymes APN et NEP, qui, en protégeant complètement les enképhalines endogènes de leur dégradation enzymatique, permettent ainsi de révéler les activités pharmacologiques, en particulier analgésiques et antidépressives, des enképhalines (Noble et al. (2007) Expert. Opin. Ther. Targets, 11, 145-149*).* Il existe déjà certains inhibiteurs mixtes de ces deux enzymes décrits dans la littérature parmi lesquels des hydroxamates (FR2518088 et FR2605004), des composés aminophosphiniques (FR2755135, FR2777780, FR0855015), des dérivés d'aminoacides à fonction thiol (FR2651229, FR0510862, FR0604030, FR0853092), des peptides endogènes (Wisner et al. (2006), PNAS, 103, 17979-17984*).* Ces différentes molécules présentent des propriétés physicochimiques (solubilité) et pharmacodynamiques (biodisponibilité) qui leur octroient une efficacité pharmacologique par voie intraveineuse ou par voie orale sur différents types de douleurs, en particulier les douleurs aigues ou chroniques par excès de nociception (Noble et al. (2007), Expert. Opin. Ther. Targets, 11, 145-149) et neuropathiques (Menendez et al. (2008), Eur J Pharmacol, 596, 50-55 ; Thibault et al. (2008), Eur. J. Pharmacol., 600, 71-77*).* M.C. Fournié-Zaluski, et al. (Journal of Médicinal Chemistry, 1985, 28, 1158) décrivent des composés utiles en tant qu'analgésiques.

L'objectif de l'invention est de fournir des composés capables de chélater efficacement les ions Zn²⁺ et d'interagir avec les différents sous-sites des enzymes NEP, et de manière avantageuse capable d'interagir également avec les sous-sites enzymatiques de l'APN et présentant les propriétés bénéfiques des substances morphiniques en particulier l'analgésie, les effets comportementaux (diminution de la composante émotionnelle de la douleur et réponses antidépressives) sans leurs inconvénients (accoutumance, dépendance physique et psychique, dépression respiratoire, constipation). D'autre part, il serait avantageux que les composés présentent des effets périphériques bénéfiques (anti-inflammatoire et douleur neuropathique) sans les inconvénients énoncés ci-dessus.

### EXPOSE DE L'INVENTION

L'invention porte sur des composés possédant la formule générale (I) suivante:

**H-CO-N(OH)-CH₂-CH(R₁)-CO-NH-(CH₂)ₙ-CH(R₂)-(CH₂)ₘ-CO-R₃** **(I)**

dans laquelle
**R₁** représente un groupement hydrocarboné linéaire ou ramifié comprenant de 1 à 6 atomes de carbone, substitué par un ou plusieurs groupements choisis parmi :
   - un aryle, lui-même non substitué ou substitué par un ou plusieurs groupements choisis parmi les halogènes tels que le fluor et le brome, un groupement phényle, un groupement benzyle, un groupement OR₄, R₄ étant choisi parmi un hydrogène et un groupement alkyle linéaire ou ramifié comprenant de 1 à 4 atomes de carbone, et leurs combinaisons,
   - un hétéroaryle à 5 ou 6 chaînons comprenant 1 ou 2 hétéroatomes, chaque hétéroatome étant choisi parmi l'oxygène, l'azote et le soufre, et
   - un cyclohétéroalkyle à 5 ou 6 chaînons comprenant 1 ou 2 hétéroatomes, chaque hétéroatome étant choisi parmi l'oxygène, l'azote et le soufre,
**R₂** représente :
   - un groupement hydrocarboné linéaire ou ramifié comprenant de 1 à 6 atomes de carbone, non substitué ou substitué par un ou plusieurs groupements choisis parmi :
      - un groupement choisi parmi OR₅, SR₅ et S(O)R₅, R₅ étant choisi parmi un hydrogène et un groupement alkyle linéaire ou ramifié comprenant de 1 à 4 atomes de carbone,
      - un groupement CO₂R₆, R₆ étant choisi parmi un hydrogène, un groupement alkyle linéaire ou ramifié comprenant de 2 à 4 atomes de carbone et un groupement benzyle,
      - un aryle, lui-même non substitué ou substitué par un ou plusieurs groupements choisis parmi les halogènes, tels que le fluor et le brome, un groupement OR₅, R₅ ayant la même définition que ci-dessus, et leurs combinaisons,
      - un hétéroaryle à 5 ou 6 chaînons comprenant 1 ou 2 hétéroatomes, chaque hétéroatome étant choisi parmi l'oxygène, l'azote et le soufre,
      - un cycloalkyle à 5 ou 6 chaînons, et
      - un cyclohétéroalkyle à 5 ou 6 chaînons comprenant 1 ou 2 hétéroatomes, chaque hétéroatome étant choisi parmi l'oxygène, l'azote et le soufre,
   - un aryle, non substitué ou substitué par un ou plusieurs groupements choisis parmi les halogènes, tels que le fluor et le brome, un groupement OR₅, R₅ ayant la même définition que ci-dessus, et leurs combinaisons, ou
   - un hétéroaryle comprenant 1 ou plusieurs hétéroatomes, de préférence 1 ou 2, chaque hétéroatome étant choisi parmi l'oxygène, l'azote et le soufre, non substitué ou substitué par un ou plusieurs groupements choisis parmi les halogènes, tels que le fluor et le brome, un groupement OR₅, R₅ ayant la même définition que ci-dessus, et leurs combinaisons,
**R₃** représente :
   - un groupement OR₇, R₇ étant choisi parmi
      - un hydrogène,
      - un groupement alkyle linéaire ou ramifié comprenant de 2 à 6 atomes de carbone,
      - un groupement benzyle, et
      - un groupement CHR₈-COOR₉, CHR₈-O-C(=O)R₉ ou CHR₈₋O-C(=O)-OR₉ dans lesquels R₈ et R₉ sont, indépendamment l'un de l'autre, choisis parmi un groupement alkyle, un groupement aryle, un groupement arylalkyle, un groupement cycloalkyle, un groupement cyclohétéroalkyle, un groupement hétéroalkyle, un groupement hétéroaryle et un groupement hétéroarylalkyle, et
m et n sont indépendamment l'un de l'autre un nombre entier choisi parmi 0 et 1, l'atome de carbone portant **R₁** ayant une configuration absolue (R) et l'atome de carbone portant **R₂** ayant une configuration absolue (S), ainsi que leurs sels et/ou solvates pharmaceutiquement acceptables.

Les composés de formule (I) de la présente invention comportent une fonction N-formyl hydroxylamine **H-CO-N(OH)-** comme ligand de Zn²⁺, capable de se comporter comme un bidentate vis-à-vis du Zn²⁺ catalytique.

Un autre objet de la présente invention concerne un composé de formule (II) suivante :

**H-CO-N(OR)-CH₂-CH(R₁)-CO-NH-(CH₂)ₙ-CH(R₂)-(CH₂)ₘ-CO-R₃** **(II)**

dans lequel :
**R** représente :
   - un groupement hydrocarboné linéaire ou ramifié de 1 à 6 atomes de carbone non substitué ou substitué par un ou plusieurs groupements aryles, eux-mêmes non substitués ou substitués par un ou plusieurs groupements choisis parmi les aryles et les groupements alkyles linéaires ou ramifiés comprenant de 1 à 4 carbones, ou
   - un groupement Si(R₁₀)₃, R₁₀ étant un groupement alkyle linéaire ou ramifié comprenant de 1 à 4 atomes de carbone, et
**R₁, R₂** et **R₃** sont tels que définis ci-dessus, l'atome de carbone portant **R₁** ayant une configuration absolue (R) et l'atome de carbone portant **R₂** ayant une configuration absolue (S),
ainsi que leurs sels et/ou solvates pharmaceutiquement acceptables.

L'invention concerne également des composés de formule (I) ou des composés de formule (II) tels que décrits ci-dessus, à condition que le groupement R dans les composés de formule (II) soit un groupement labile dans les conditions physiologiques, pour leur utilisation en tant que médicament, en particulier en tant qu'analgésique, anxiolytique, antidépresseur ou anti-inflammatoire.

L'invention porte également sur des compositions pharmaceutiques comprenant au moins un composé de formule (I) ou des composés de formule (II) selon la présente invention, à condition que le groupement R dans les composés de formule (II) soit un groupement labile dans les conditions physiologiques.

L'invention porte également sur des compositions pharmaceutiques comprenant au moins un composé de formule (I) ou des composés de formule (II) tels que décrits ci-dessus, à condition que le groupement R dans les composés de formule (II) soit un groupement labile dans les conditions physiologiques, et au moins un composé choisi parmi la morphine et ses dérivés, les endocannabinoïdes et les inhibiteurs de leur métabolisme, les dérivés du GABA tels que la gabapentine ou la prégabaline, la duloxetine ou les inhibiteurs des canaux tels que les inhibiteurs de Nav 1,7. L'invention concerne les compositions pharmaceutiques telles que décrites ci-dessus pour leur utilisation en tant qu'analgésique, anxiolytique, antidépresseur ou anti-inflammatoire.

### DESCRIPTION DES FIGURES

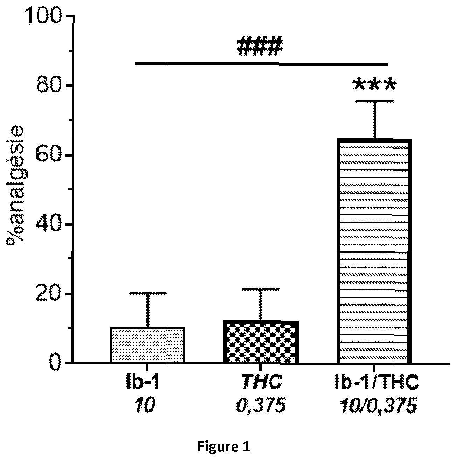

La figure 1 illustre l'effet de la coadministration intraveineuse de THC et du composé **Ib-1** solubilisé dans (EtOH/Tween 80/Eau) (1/1/8) comme véhicule dans un test de plaque chaude chez la souris. La barre grisée correspond à la réponse analgésique (10%) suite à l'injection du composé **Ib-1** uniquement (à hauteur de 10 mg/kg), la barre damée correspond à la réponse analgésique (12%) suite à l'injection de THC uniquement (à hauteur de 0,375 mg/kg) et la barre comprenant des lignes horizontales correspond à la réponse analgésique (65%) suite à l'injection du composé **Ib-1** en association avec le composé **Ib-1**(à hauteur de 10 mg/kg de **1b-1** pour 0,375 mg/kg de THC).

### DESCRIPTION DETAILLEE DE L'INVENTION

Dans le cadre de la présente invention, l'expression « groupement hydrocarboné » désigne un groupement alkyle, un groupement alcényle ou un groupement alcynyle tel que défini ci-après. Par « groupement alkyle », on entend, au sens de la présente invention, une chaîne hydrocarbonée saturée, linéaire ou ramifiée. A titre d'exemple, on peut citer les groupements méthyle, éthyle, propyle, isopropyle, butyle, iso-butyle, sec-butyle, *tert*-butyle, pentyle ou encore hexyle.

Par « groupement alcényle » on entend, au sens de la présente invention, une chaîne hydrocarbonée, linéaire ou ramifiée, comprenant une ou plusieurs double liaisons. A titre d'exemple, on peut citer les groupements éthenyle, propényle, butényle, pentényle ou encore hexényle.

Par « groupement alcynyle », on entend, au sens de la présente invention, une chaîne hydrocarbonée, linéaire ou ramifiée, comportant au moins une triple liaison. A titre d'exemple, on peut citer les groupements éthynyle ou propynyle.

Au sens de la présente invention, le groupement hydrocarboné comprend avantageusment de 1 à 6 atomes de carbone s'il s'agit d'un groupement alkyle et de 2 à 6 atomes de carbones s'il s'agit d'un groupement alcényle ou alcynyle.

Par « cycloalkyle », on entend, au sens de la présente invention, un cycle hydrocarboné saturé comportant avantageusement 5 ou 6 atomes de carbone, en particulier le groupe cyclohexyle ou cyclopentyle.

Par « cyclohétéroalkyle », on entend au sens de la présente invention, un cycle hydrocarboné saturé comportant avantageusement 5 ou 6 atomes de carbone dans lequel un ou plusieurs atomes de carbone, avantageusement 1 à 2, sont chacun remplacés par un hétéroatome choisi parmi les atomes de soufre, d'azote et d'oxygène.

Par « hétéroalkyle », on entend au sens de la présente invention un groupement alkyle tel que défini ci-dessus dans lequel un ou plusieurs atomes de carbone, avantageusement 1 à 2, sont chacun remplacés par un hétéroatome choisi parmi les atomes de soufre, d'azote et d'oxygène.

Par « aryle », on entend, au sens de la présente invention, un groupement hydrocarboné aromatique, comportant de préférence de 6 à 10 atomes de carbone et comprenant un ou plusieurs cycles accolés. Il s'agira avantageusement d'un groupement phényle ou naphtyle, de préférence un phényle.

Par « arylalkyle », on entend, au sens de la présente invention, un groupement alkyle tel que défini ci-dessus, dans lequel un ou plusieurs atomes d'hydrogène, de préférence 1 ou 2, portés par le même carbone ou par plusieurs atomes de carbone différents, sont remplacés par un groupement aryle tel que défini ci-dessus. Il s'agira avantageusement d'un groupement benzyle.

Par « hétéroaryle », on entend, au sens de la présente invention, un groupe aromatique comprenant avantageusement 5 ou 6 atomes, dans lequel un ou plusieurs atomes de carbone, avantageusement 1 à 2, sont chacun remplacés par un hétéroatome choisi parmi les atomes de soufre, d'azote et d'oxygène. Des exemples de groupes hétéroaryle sont les groupes furyle, thiényle, pyrrolyle, oxazolyle, isoxazolyle, thiazolyle, isothiazolyle, imidazolyle, pyrazolyle, oxadiazolyle, thiadiazolyle, triazolyle, tétrazolyle, pyridyle, pyridazinyle, pyrimidinyle, pyrazinyle, triazinyle, quinolyle, quinoxalyle ou encore indolyle. De préférence, il s'agit d'un groupe thiényle, en tant qu'isostére du groupement phényl.

Par « hétéroarylalkyle », on entend au sens de la présente invention un groupement alkyle tel que défini ci-dessus, dans lequel un ou plusieurs atomes d'hydrogène, de préférence 1 ou 2, portés par le même carbone ou par plusieurs atomes de carbone différents, sont remplacés par un groupement hétéroaryle tel que défini ci-dessus.

Le terme « halogène » désigne un chlore, un brome, un iode et un fluor. Avantageusement, il s'agit d'un atome de fluor, de brome ou de chlore. Encore avantageusement, il s'agit d'un atome de fluor ou de brome, et de préférence de fluor. Par « insaturé », on entend, au sens de la présente invention, que la chaîne hydrocarbonée peut comporter une ou plusieurs insaturation(s), avantageusement une. Par « insaturation », on entend, au sens de la présente invention, une double ou une triple liaison carbone-carbone (C=C ou C=C).

Par « stéréoisomère », on entend, au sens de la présente invention, un isomère géométrique ou un isomère optique.

Les isomères géométriques résultent de la position différente des substituants sur une double liaison qui peut avoir alors une configuration Z ou E.

Les isomères optiques résultent notamment de la position différente dans l'espace des substituants sur un atome de carbone comprenant 4 substituants différents. Cet atome de carbone constitue alors un centre chiral ou asymétrique. Les isomères optiques comprennent les diastéréoisomères et les énantiomères. Les isomères optiques qui sont des images l'un de l'autre dans un miroir mais non superposables sont désignés par « énantiomères ». Les isomères optiques qui ne sont ni superposables, ni images l'un de l'autre dans un miroir sont désignés par « diastéréoisomères ».

Un mélange contenant des quantités égales de deux formes énantiomères individuelles de chiralité opposée est désigné par « mélange racémique ».

Par « groupe chiral », on entend, au sens de la présente invention, un groupe qui n'est pas superposable à son image dans un miroir. Un tel groupe chiral pourra comprendre en particulier un atome de carbone asymétrique, c'est-à-dire un atome de carbone substitué par quatre substituants (incluant l'hydrogène) différents.

Par « configuration absolue », on entend, au sens de la présente invention, l'arrangement spatial des atomes ou groupes chimiques autour de l'atome de carbone asymétrique auquel ces atomes et groupes chimiques sont liés. Les deux configurations absolues possibles d'un carbone asymétrique sont notées S et R.

Par « synthèse énantiosélective », on entend au sens de la présente invention, une synthèse conduisant à l'obtention d'un seul énantiomère de la molécule synthétisée.

Le terme « réaction de N-formylation » désigne une réaction au cours de laquelle un atome d'azote d'un composé organique est substitué par un groupement formyle -CHO. Le terme « agent de couplage peptidique » désigne un réactif organique capable d'activer la fonction acide carboxylique d'un composé organique fin que cette dernière puisse former une liaison peptidique avec une fonction amine terminale d'un autre composé organique. Parmi les agents de couplage les plus communément utilisés, on trouve HATU (hexafluorophosphate de (diméthylamino)-N,N-diméthyl(3H-[1,2,3]triazolo[4,5-b]pyridin-3-yloxy)méthaniminium), TBTU (tetrafluoroborate de 2-(1H-benzotriazole-1-yl)-1,1,3,3-tetramethylaminium), BOP (hexafluorophosphate de benzotriazol-1-yloxytris(dimethylamino)phosphonium), PyBOP (hexafluorophosphate de benzotriazol-1-yl-oxytripyrrolidinophosphonium), HOBt (hydroxybenzotriazole) ou encore les carbodiimides, tel que le DCC (dicyclohexylcarbodiimide) et EDC (1-éthyl-3-(3-diméthylaminopropyl)carbodiimide). Ces agents de couplage peuvent parfois être utilisés en combinaison avec d'autres. Par exemple, l'EDC est souvent utilisé en combinaison avec HOBt dans les réactions de couplage peptidique.

Par « groupement labile dans les conditions physiologiques », on entend, au sens de la présente invention, un groupement chimique, généralement un groupement protecteur par exemple d'une fonction hydroxyle, amine ou acide, qui sera retiré dans les conditions physiologiques lors de leur pénétration dans le corps. Par exemple, lors d'une administration orale, le pH acide de l'estomac pourra être responsable de la déprotection dudit groupement labile.

Les composés de l'invention peuvent être sous forme de sels pharmaceutiquement acceptables, ou d'un solvate de ceux-ci. Dans la présente invention, on entend par « pharmaceutiquement acceptable » ce qui est utile dans la préparation d'une composition pharmaceutique qui est généralement sûr, non toxique et qui est acceptable pour une utilisation vétérinaire de même que pharmaceutique humaine.

On entend désigner par « sels pharmaceutiquement acceptables » d'un composé, des sels qui sont pharmaceutiquement acceptables, comme défini ici, et qui possèdent l'activité pharmacologique souhaitée du composé parent. De tels sels comprennent :
(1) les sels d'addition pharmaceutiquement acceptable formés avec des bases, et
(2) les hydrates et les solvates de ceux-ci.

Typiquement, les composés de formule (I) sont sous forme de sels d'addition obtenus avec des bases organiques ou inorganiques pharmacologiquement acceptables ou avec un ion métallique, tel qu'un ion métallique alcalin ou alcalino-terreux. Des bases organiques sont par exemples la diéthanolamine, l'éthanolamine, la N-méthylglucamine, la triéthanolamine et la trométhamine. Des bases inorganiques sont par exemple l'hydroxyde d'aluminium, l'hydroxyde de calcium, l'hydroxyde de potassium, le carbonate de sodium et l'hydroxyde de sodium. Les solvates acceptables pour l'utilisation thérapeutique des composés de l'invention incluent les solvates conventionnels tels que ceux formés durant la dernière étape de la préparation des composés en raison de la présence éventuelle de solvants. Par exemple, il peut s'agir de solvates dus à la présence d'eau (qu'on appelle hydrates) ou d'éthanol. Le solvate est de préférence un alcoolate, tel qu'un éthanolate.

De préférence, les composés de l'invention sont sous forme de sels de sodium ou d'un hydrate de ceux-ci.

**R₁** représente avantageusement un groupement hydrocarboné linéaire ou ramifié comprenant de 1 à 6 atomes de carbone substitué par un ou plusieurs groupements choisis parmi :
- un aryle, lui-même non substitué ou substitué par un ou plusieurs groupements choisis parmi les halogènes tels que le fluor et le brome, un groupement phényle, un groupement benzyle, un groupement OR₄, R₄ étant choisi parmi un hydrogène et un groupement alkyle linéaire ou ramifié comprenant de 1 à 4 atomes de carbone, et leurs combinaisons,
- un hétéroaryle à 5 ou 6 chaînons comprenant 1 ou 2 hétéroatomes, chaque hétéroatome étant choisi parmi l'oxygène, l'azote et le soufre,
- un cyclohétéroalkyle à 5 ou 6 chaînons comprenant 1 ou 2 hétéroatomes, chaque hétéroatome étant choisi parmi l'oxygène, l'azote et le soufre.

**R₁** représente avantageusement un groupement hydrocarboné linéaire ou ramifié comprenant de 1 à 6 atomes de carbone, substitué par un aryle, lui-même non substitué ou substitué par un ou plusieurs groupements choisis parmi les halogènes tels que le fluor et le brome, un groupement phényle, un groupement benzyle, un groupement OR₄, R₄ étant choisi parmi un hydrogène et un groupement alkyle linéaire ou ramifié comprenant de 1 à 4 atomes de carbones, et leurs combinaisons. Selon un mode de réalisation préféré, **R₁** représente un groupement hydrocarboné linéaire ou ramifié comprenant de 1 à 6 atomes de carbone, substitué par un aryle, lui-même non substitué ou substitué par un ou plusieurs groupements choisis parmi les halogènes tels que le fluor et le brome, un groupement phényle, un groupement benzyle, un groupement OR₄, R₄ étant choisi parmi un hydrogène et un groupement alkyle linéaire ou ramifié comprenant de 1 à 4 atomes de carbones, et leurs combinaisons. Plus avantageusement, **R₁** représente un groupement hydrocarboné, de préférence un groupement alkyle linéaire ou ramifié comprenant de 1 à 6 atomes de carbone, de préférence 1 atome de carbone, substitué par un aryle, de préférence un groupement phényle, lui-même non substitué ou substitué par un groupement phényle.

**R₂** représente avantageusement :
- un groupement hydrocarboné linéaire ou ramifié comprenant de 1 à 6 atomes de carbone, non substitué ou substitué par un ou plusieurs groupements choisis parmi :
   - un groupement choisi parmi OR₅, SR₅ et S(O)R₅, R₅ étant choisi parmi un hydrogène et un groupement alkyle linéaire ou ramifié comprenant de 1 à 4 atomes de carbone,
   - un groupement CO₂R₆, R₆ étant choisi parmi un hydrogène, un groupement alkyle linéaire ou ramifié comprenant de 2 à 4 atomes de carbone et un groupement benzyle,
   - un aryle, lui-même non substitué ou substitué par un ou plusieurs groupements choisis parmi les halogènes, tels que le fluor et le brome, un groupement OR₅, R₅ ayant la même définition que ci-dessus, et leurs combinaisons,
   - un hétéroaryle à 5 ou 6 chaînons comprenant 1 ou 2 hétéroatomes, chaque hétéroatome étant choisi parmi l'oxygène, l'azote et le soufre,
   - un cycloalkyle à 5 ou 6 chaînons, et
   - un cyclohétéroalkyle à 5 ou 6 chaînons comprenant 1 ou 2 hétéroatomes, chaque hétéroatome étant choisi parmi l'oxygène, l'azote et le soufre.

**R₂** représente:
- un groupement hydrocarboné linéaire ou ramifié comprenant de 1 à 6 atomes de carbone, non substitué ou substitué par un ou plusieurs groupements choisis parmi :
   - un groupement choisi parmi OR₅, SR₅ et S(O)R₅, R₅ étant choisi parmi un hydrogène et un groupement alkyle linéaire ou ramifié comprenant de 1 à 4 atomes de carbone,
   - un groupement CO₂R₆, R₆ étant choisi parmi un hydrogène, un groupement alkyle linéaire ou ramifié comprenant de 2 à 4 atomes de carbone et un groupement benzyle,
   - un aryle, lui-même non substitué ou substitué par un ou plusieurs groupements choisis parmi les halogènes, tels que le fluor et le brome, un groupement OR₅, R₅ ayant la même définition que ci-dessus, et leurs combinaisons,
   - un hétéroaryle à 5 ou 6 chaînons comprenant 1 ou 2 hétéroatomes, chaque hétéroatome étant choisi parmi l'oxygène, l'azote et le soufre,
   - un cycloalkyle à 5 ou 6 chaînons, et
   - un cyclohétéroalkyle à 5 ou 6 chaînons comprenant 1 ou 2 hétéroatomes, chaque hétéroatome étant choisi parmi l'oxygène, l'azote et le soufre,
- un aryle, non substitué ou substitué par un ou plusieurs groupements choisis parmi les halogènes, tels que le fluor et le brome, un groupement OR₅, R₅ ayant la même définition que ci-dessus, et leurs combinaisons, ou
- un hétéroaryle comprenant 1 ou plusieurs hétéroatomes, de préférence 1 ou 2, chaque hétéroatome étant choisi parmi l'oxygène, l'azote et le soufre, non substitué ou substitué par un ou plusieurs groupements choisis parmi les halogènes, tels que le fluor et le

brome, un groupement OR₅, R₅ ayant la même définition que ci-dessus, et leurs combinaisons.

Selon un mode de réalisation préféré, **R₂** représente un groupement hydrocarboné linéaire ou ramifié comprenant de 1 à 6 atomes de carbone, non substitué ou substitué par un ou plusieurs groupements choisis parmi :
- un groupement choisi parmi OR₅, SR₅ et S(O)R₅, R₅ étant choisi parmi un hydrogène et un groupement alkyle linéaire ou ramifié comprenant de 1 à 4 atomes de carbone,
- un groupement CO₂R₆, R₆ étant choisi parmi un hydrogène, un groupement alkyle linéaire ou ramifié comprenant de 2 à 4 atomes de carbone et un groupement benzyle,
- un aryle, lui-même non substitué ou substitué par un ou plusieurs groupements choisis parmi les halogènes, tels que le fluor et le brome, un groupement OR₅, R₅ ayant la même définition que ci-dessus, et leurs combinaisons,
- un hétéroaryle à 5 ou 6 chaînons comprenant 1 ou 2 hétéroatomes, chaque hétéroatome étant choisi parmi l'oxygène, l'azote et le soufre,
- un cycloalkyle à 5 ou 6 chaînons, et
- un cyclohétéroalkyle à 5 ou 6 chaînons comprenant 1 ou 2 hétéroatomes,
chaque hétéroatome étant choisi parmi l'oxygène, l'azote et le soufre.

Plus avantageusement, **R₂** représente un groupement hydrocarboné, de préférence un groupement alkyle, linéaire ou ramifié comprenant de 1 à 6 atomes de carbone, non substitué ou substitué par un ou plusieurs groupements choisis parmi :
- un aryle, lui-même non substitué ou substitué par un ou plusieurs groupements choisis parmi les halogènes, tels que le fluor et le brome, un groupement OR₅, R₅ ayant la même définition que ci-dessus, et leurs combinaisons,
- un cycloalkyle à 5 ou 6 chaînons, et
- un cyclohétéroalkyle à 5 ou 6 chaînons comprenant 1 ou 2 hétéroatomes,
chaque hétéroatome étant choisi parmi l'oxygène, l'azote et le soufre.

En particulier, **R₂** représente un groupement hydrocarboné, de préférence un groupement alkyle, linéaire ou ramifié comprenant de 1 à 6 atomes de carbone, non substitué ou substitué par un aryle, lui-même non substitué ou substitué par un ou plusieurs groupements choisis parmi les halogènes, tels que le fluor et le brome, un groupement OR₅, R₅ ayant la même définition que ci-dessus, et leurs combinaisons. Plus particulièrement, le radical **R₂** représente un groupement hydrocarboné, de préférence un groupement alkyle, linéaire ou ramifié comprenant de 1 à 6 atomes de carbone, non substitué ou substitué par un aryle, lui-même non substitué. De préférence, le groupement aryle est un groupement phényle. De manière plus avantageusement, le radical **R₂** représente un groupement alkyle, linéaire ou ramifié comprenant de 1 à 6 atomes de carbone, de préférence 1 atome de carbone, non substitué ou substitué par un phényle.

**R₃** représente avantageusement un groupement OR₇, R₇ étant choisi parmi :
- un hydrogène,
- un groupement alkyle linéaire ou ramifié comprenant de 2 à 6 atomes de carbone, et
- un groupement benzyle.

Selon un mode de réalisation préféré, **R₃** représente un groupement OR₇, R₇ étant choisi parmi:
- un hydrogène, et
- un groupement alkyle linéaire ou ramifié comprenant de 2 à 6 atomes de carbone.

De manière encore plus préférée, **R₃** représente un groupement OH.

Selon un mode de réalisation particulier, au sein du composé de formule (I), **R₁** représente un groupement hydrocarboné, notamment un groupement alkyle, linéaire ou ramifié comprenant de 1 à 6 atomes de carbone, substitué par un aryle, de préférence un phényle, lui-même non substitué ou substitué par un ou plusieurs groupements choisis parmi le fluor, le brome, un groupement phényle, un groupement benzyle, un groupement OR₄, R₄ étant choisi parmi un hydrogène et un groupement alkyle linéaire ou ramifié comprenant de 1 à 4 atomes de carbone, et leurs combinaisons, de préférence par un groupement phényle,

**R₂** représente un groupement hydrocarboné, de préférence un groupement alkyle, linéaire ou ramifié de 1 à 6 atomes de carbone, non substitué ou substitué, par un ou plusieurs groupements choisis parmi :
- un aryle, lui-même non substitué ou substitué par un ou plusieurs groupements choisis parmi le fluor, le brome, un groupement OR₅, R₅ étant choisi parmi un hydrogène et un groupement alkyle linéaire ou ramifié comprenant de 1 à 4 atomes de carbone, et leurs combinaisons,
- un hétéroaryle à 5 ou 6 chaînons comprenant 1 ou 2 hétéroatomes, chaque hétéroatome étant choisi parmi l'oxygène, l'azote et le soufre,
- un cycloalkyle à 5 ou 6 chaînons, et
- un cyclohétéroalkyle à 5 ou 6 chaînons comprenant 1 ou 2 hétéroatomes, chaque hétéroatome étant choisi parmi l'oxygène, l'azote et le soufre,
de préférence, **R₂** représente un groupement alkyle, linéaire ou ramifié de 1 à 6 atomes de carbone, non substitué ou substitué par un phényle, et

**R₃** représente un groupement OR₇, R₇ étant choisi parmi :
- un hydrogène, et
- un groupement alkyle linéaire ou ramifié comprenant de 2 à 6 atomes de carbone,
de préférence R₇ est un hydrogène.

Selon un mode de réalisation particulier, le composé de formule (I) selon la présente invention est un composé dans lequel :
- **R₁** = (R)-CH₂Ph ; **R₂** = (S)-CH₃ ; **R₃** = OH ; n=m=0;
- **R₁** = (S)-CH₂Ph ; **R₂** = (S)-CH₃ ; **R₃** = OH ; n=m=0;
- **R₁** = (R)-CH₂Ph ; **R₂** = (S)-CH₂Ph ; **R₃** = OH ; n=m=0;
- R**₁** = (S)-CH₂Ph ; **R₂** = (S)-CH₂Ph ; **R₃** = OH ; n=m=0;
- R**₁** = (R)-CH₂Ph ; **R₂** = (S)-CH₂Ph ; **R₃** = OH ; n=0; m=1;
- **R₁** = (S)-CH₂Ph ; **R₂** = (S)-CH₂Ph ; **R₃** = OH ; n=0; m=1;
- **R₁** = (R)-CH₂Ph-4-Ph ; **R₂** = (S)-CH₃ ; **R₃** = OH ; n=m=0;
- **R₁** = (S)-CH₂Ph-4-Ph ; **R₂** = (S)-CH₃ ; **R₃** = OH ; n=m=0;
- **R₁** = (S)-CH₂Ph ; **R₂** = (S)-CH₂Ph ; **R₃** = OH ; n=1; m=0;
- **R₁** = (R)-CH₂Ph ; **R₂** = (S)-CH₂Ph ; **R₃** = OH ; n=1; m=0,
ou un sel et/ou un solvate pharmaceutiquement acceptable de celui-ci.

De manière préférée, le composé de l'invention est
- **R₁** = (R)-CH₂Ph ; **R₂** = (S)-CH₃ ; **R₃** = OH ; n=m=0;
- **R₁** = (R)-CH₂Ph ; **R₂** = (S)-CH₂Ph ; **R₃** = OH ; n=m=0;
- **R₁** = (R)-CH₂Ph ; **R₂** = (S)-CH₂Ph ; **R₃** = OH ; n=0; m=1;
- **R₁** = (R)-CH₂Ph-4-Ph ; **R₂** = (S)-CH₃ ; **R₃** = OH ; n=m=0;
- **R₁** = (R)-CH₂Ph ; **R₂** = (S)-CH₂Ph ; **R₃** = OH ; n=1; m=0,

La présente invention concerne également un composé de formule (II) suivante :

**H-CO-N(OR)-CH₂-CH(R₁)-CO-NH-(CH₂)ₙ-CH(R₂)-(CH₂)ₘ-CO-R₃** **(II)**

dans lequel :
**R** représente :
   - un groupement hydrocarboné linéaire ou ramifié comprenant de 1 à 6 atomes de carbone non substitué ou substitué par un ou plusieurs groupements aryles, eux-mêmes non substitués ou substitués par un ou plusieurs groupements choisis parmi les aryles et les groupements alkyles linéaires ou ramifiés comprenant de 1 à 4 carbones, ou
   - un groupement Si(R₁₀)₃, R₁₀ étant un groupement alkyle linéaire ou ramifié comprenant de 1 à 4 atomes de carbone, et
**R₁, R₂, R₃,** m et n sont tels que définis ci-dessus,
   l'atome de carbone portant **R₁** ayant une configuration absolue (R) et l'atome de carbone portant **R₂** ayant une configuration absolue (S),
   ainsi que leurs sels et/ou solvates pharmaceutiquement acceptables.

De préférence, **R** représente un groupement alkyle linéaire ou ramifié comprenant de 1 à 6 atomes de carbone non substitué ou substitué par un groupement aryle, de préférence un phényle, ou **R** représente un groupement Si(R₁₀)₃, R₁₀ étant préférablement un méthyle. De manière plus préférée, **R** représente un groupement benzyle.

Lorsque R est un groupement chimique labile tel que défini précédemment, le composé (II) se transforme en composé (I) lors de sa pénétration dans le corps sous l'action des conditions physiologiques adaptées à la déprotection du groupement R. Le composé (II) constitue alors une prodrogue du composé (I).

Les composés de la présente invention de formule (I) ou de formule (II), à la condition que le groupement R soit un groupement labile sous conditions physiologiques peuvent être utilisés en tant que médicament. Plus particulièrement, ces composés peuvent être employés pour préparer des compositions pharmaceutiques comprenant à titre de principe actif au moins un des composés décrits ci-dessus en combinaison avec au moins un excipient pharmaceutiquement acceptable. Lesdits excipients sont choisis selon la forme pharmaceutique et le mode d'administration souhaités parmi les excipients habituels qui sont connus de l'homme du métier.

Les composés de la présente invention inhibant conjointement les activités enzymatiques responsables de la dégradation des enképhalines, ils augmentent leur taux endogène extracellulaire et s'avèrent à ce titre être des analgésiques et/ou antidépresseurs efficaces. Les effets analgésiques des composés se manifestent sur diverses douleurs, aiguës ou chroniques, telles que les douleurs post-opératoires, cancéreuses, traumatologiques, les céphalées, les migraines, les douleurs viscérales, neurogéniques, neuropathiques, neuro-inflammatoires, nociceptives ou générale comme la fibromyalgie. Des exemples de douleur incluent les douleurs mécaniques (par exemple douleur musculaire, ischémie d'origine vasculaire), les douleurs occasionnées par un zona, les douleurs cancéreuses liées au cancer lui-même ou aux conséquences des traitements, les douleurs associées aux maladies inflammatoires ou dégénératives (par exemple, arthrite, polyarthrite rhumatoïde, arthrose, goutte), les douleurs liées au diabète de type 1 et 2, les douleurs liées aux migraines, aux névralgie faciales, les céphalées, les douleurs liées aux atteintes des nerfs périphériques, les névralgies dorso-lombaires, cervico-brachiales, les douleurs dentaires, les douleurs liées aux brûlures, coups de soleil, morsures ou piqûres, les douleurs liées aux infections, troubles métaboliques (diabète, alcoolisme), aux compressions nerveuses (hernies discales, canal carpien, fibrose...), fractures, brûlures, hématomes, coupures et inflammation, les douleurs viscérales (intestinales, causées par les maladies inflammatoires de l'intestin et les troubles fonctionnels intestinaux, par les cholécystites urinaires, comme les coliques néphrétiques, les cystites, génitales, comme les dysménorrhées, les cystites, l'endométriose cardiaques, comme les douleurs de l'infarctus du myocarde).

Enfin, typiquement, et de manière avantageuse, les composés de la présente invention ne présentent pas les inconvénients majeurs des substances morphiniques (tolérance, dépendance physique, dépression respiratoire, nausée, sédation, constipation, ...).

Ainsi, les composés de la présente invention de formule (I) ou de formule (II), à la condition que le groupement R soit un groupement labile sous conditions physiologiques et les compositions pharmaceutiques les renfermant peuvent utiles pour au moins une utilisation choisie parmi les utilisations suivantes : analgésique, anxiolytique, antidépresseur ou anti-inflammatoire.

La présente invention concerne également l'utilisation des composés de formule (I) ou de formule (II), tels que définis ci-dessus, à la condition que le groupement R soit un groupement labile sous conditions physiologiques et les compositions pharmaceutiques les renfermant pour la fabrication d'un médicament analgésique, anxiolytique, antidépresseur ou anti-inflammatoire, plus particulièrement d'un médicament destiné au traitement de la douleur. La douleur peut être notamment une douleur chronique ou aiguë telle que définie ci-dessus.

La présente invention concerne également une méthode de traitement de la douleur, notamment une douleur chronique ou aiguë telle que définie ci-dessus, comprenant l'administration à un patient en ayant besoin d'une dose efficace du composé de formule (I) ou de formule (II) selon l'invention, à la condition que le groupement R soit un groupement labile sous conditions physiologiques ou un de leurs sels et/ou solvates pharmacetiquement acceptable ou d'une composition selon l'invention, de préférence par voie parentérale, voie orale ou nasale.

Dans la présente invention, le patient (souffrant de douleurs, notamment une douleur chronique ou aigüe telle que définie ci-dessus) est typiquement un animal, de préférence un mammifère, avantageusement il s'agit d'un homme.

Les composés de la présente invention peuvent être utilisés seuls ou en combinaison avec des composés connus pour leurs propriétés antinociceptives. Cette combinaison peut permettre une potentialisation des effets pharmacologiques, d'autant que les composés antinociceptifs connus présentent généralement à fortes doses des effets secondaires indésirables.

De telles potentialisations (synergies) des effets pharmacologiques ont été démontrées dans le passé en combinant des inhibiteurs mixtes présentant une structure chimique différente de celle des inhibiteurs mixtes de la présente invention avec des composés antinociceptifs connus. Ainsi, une forte potentialisation des réponses antinociceptives a été obtenue, par exemple, par combinaison avec : la morphine (Mas Nieto et al. (2001), Neuropharmacol. 41, 496-506, le THC (Valverde et al. (2001), Eur. J. Neurosci., 13, 1816-1824), la gabapentine (Menendez et al. (2007), Eur. J. Pharmacol., 596, 50-55) et ses analogues tels que la prégabaline. Ces associations permettent pour un effet pharmacologique équivalent de réduire de 3 à 10 fois les doses des composants de l'association (morphine et inhibiteur par exemple).

Ainsi, dans un mode de réalisation, les compositions pharmaceutiques comprennent à titre de principe actif au moins un des composés de la présente invention en combinaison avec au moins un antinociceptif et au moins un excipient pharmaceutiquement acceptable. Les antinociceptifs peuvent être choisis parmi :
- la morphine et ses dérivés,
- les endocannabinoïdes et les inhibiteurs de leur métabolisme, le Δ⁹ THC, les agonistes des récepteurs cannabinoïdes synthétiques ou les inhibiteurs de la dégradation de l'anandamide (FAAH),
- les dérivés du GABA, tels que la gabapentine ou la prégabaline,
- la duloxetine, inhibiteur de la recapture de la sérotonine et de la noradrénaline, ou
- les inhibiteurs des canaux tels que les inhibiteurs de Nav 1,7.

Selon un mode de réalisation, les compositions pharmaceutiques de la présente invention comprennent à titre de principe actif au moins un des composés de la présente invention en combinaison avec du THC. Dans un autre mode de réalisation, la présente invention concerne un kit comprenant:
a) une première composition comprenant au moins un composé de formule (I) ou de formule (II), tels que défini ci-dessus, à la condition que le groupement R soit un groupement labile sous conditions physiologiques , et
b) une seconde composition comprenant au moins un autre principe actif, notamment un analgésique, choisi parmi la morphine et ses dérivés, les endocannabinoïdes et les inhibiteurs de leur métabolisme, les dérivés du GABA, tels que la gabapentine ou la prégabaline, la duloxetine ou les inhibiteurs des canaux tels que les inhibiteurs de Nav 1,7,
en tant que produit de combinaison pour une utilisation simultanée, séparée ou étalée dans le temps.

Le kit selon présente invention est notamment utilisé en tant qu'analgésique, anxiolytique, antidépresseur ou anti-inflammatoire, en particulier pour le traitement de la douleur, typiquement des douleurs chroniques ou aiguës telle que définies ci-dessus.

Les compositions pharmaceutiques selon l'invention peuvent être administrées par voie parentérale, telle que par voie intraveineuse ou intradermique, ou par voie topique, orale ou nasale.

Les formes administrables par voie parentérale incluent les suspensions aqueuses, les solutions salines isotoniques ou les solutions stériles et injectables qui peuvent contenir des agents de dispersion et/ou des mouillants pharmacologiquement compatibles. Les formes administrables par voie orale incluent les comprimés, les gélules molles ou dures, les poudres, les granules, les solutions et suspensions orales. Les formes administrables par voie nasale incluent les aérosols. Les formes administrables par voie topique incluent les patchs, les gels, les crèmes, les pommades, les lotions, les sprays, les collyres.

La dose efficace d'un composé de l'invention varie en fonction de nombreux paramètres tels que, par exemple, la voie d'administration choisie, le poids, l'âge, le sexe, l'état d'avancement de la pathologie à traiter et la sensibilité de l'individu à traiter.

La présente invention concerne également une méthode de préparation d'un composé de formule (I) et d'un composé de formule (II) selon la présente invention, ladite méthode comprenant les étapes successives suivantes :
(a) réaction d'un composé de formule (III) suivante :

   **H-CO-N(OR)-CH₂-CH(R₁)-C(O)OH** **(III),**

   avec un composé de formule (IV) suivante :

   **H₃N⁺-(CH₂)ₙ-CH(R₂)-(CH₂)ₘ-C(O)R₃** **(IV)**

   dans lesquels **R, R₁, R₂, R₃,** n et m sont tels que définis ci-dessus, en présence d'un agent de couplage peptidique, tel que TBTU, HATU, EDC, HOBt, BOP, PyBOP, DCC ou leurs combinaisons, conduisant à la formation d'un composé de formule (II) tel que défini ci-dessus,
(b) déprotection du composé de formule (II) issus de l'étape (a) pour conduire à un composé de formule (I).

Les composés de formule (I) ont potentiellement de 1 à 3 centres d'asymétrie. Les radicaux R₁, R₂ et R₃ seront typiquement introduits de façon à obtenir des enchaînements optiquement purs correspondant à une stéréochimie reconnue pour les interactions avec les sites actifs des enzymes concernées.

### Etape a :

Selon un mode de réalisation particulier, le composé de formule **II** peut être obtenu à l'aide des étapes de synthèse suivantes :
   **(i-1)** Réaction d'un dérivé d'acide acrylique **V** avec une hydroxylamine **VI** pour conduire à l'acide **VII**, 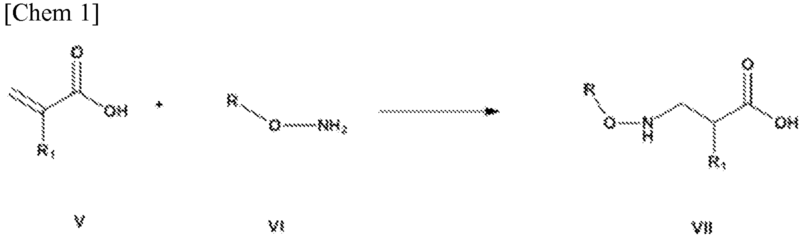
où **R** et R**₁** son tels que définis précédemment.

De préférence, l'hydroxylamine **VI** correspond à la benzylhydroxylamine.

**(i-2)** Réaction de N-formylation de l'acide **VII** dans l'acide formique en présence d'anhydride acétique pour donner le composé de formule **III**,

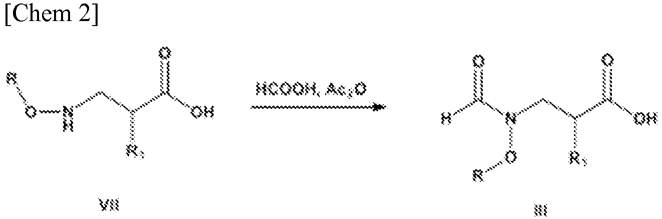

Le composé **III** subit ensuite un couplage peptidique avec le composé de formule **IV** pour conduire au composé de formule **II**: 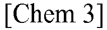 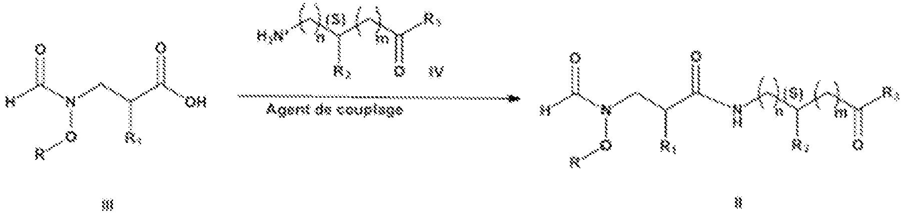 où **R, R₁, R₂, R₃,** n et m sont tels que définis ci-dessus.

De préférence, l'agent de couplage utilisé est le TBTU. Avantageusement, la réaction est réalisée en présence de DIEA (diisopropyléthylamine) dans un solvant aprotique polaire, tel que le DMF.

Selon un mode de réalisation préféré, le composé **IV** est énantiomériquement pur. Le carbone portant le groupement **R₂** a une configuration absolue résolue et correspond avantageusement à la configuration absolue (S).

Selon une variante, la méthode de préparation d'un composé de formule **1** comprend les étapes suivantes :
(a') réaction d'un composé de formule **III** tel que défini ci-dessus avec un composé de formule **VIII** suivante :

   **H₃N⁺-(CH₂)ₙ-CH(R₂)-(CH₂)ₘ-C(O)OP** **(VIII)**
dans laquelle **R₂** est tel que défini ci-dessus et OP est un précurseur de **R₃,**
**P** peut par exemple être identique à **R.** En particulier, **P** peut être un groupement benzyle, conduisant à la formation d'un composé de formule **IX** suivante :

   **H-CO-N(OR)-CH₂-CH(R₁)-CO-NH-(CH₂)ₙ-CH(R₂)-(CH₂)ₘ-C(O)-OP** **(IX)**
dans laquelle **R, R₁, R₂, P,** n et m sont tels que définis ci-dessus,
(b') transformation du groupement OP du composé **IX** en groupement R₃ tel que défini ci-dessus pour conduire au composé de formule **II** ou de formule **I** tels que définis ci-dessus.
(c') éventuellement, déprotection du composé de formule **II** issus de l'étape (b') pour conduire à un composé de formule **I**.

L'étape (b') de transformation du groupement **OP** précurseur de **R₃** en groupement **R₃** se fait selon des méthodes bien connues de l'homme du métier. Il s'agit, par exemple, d'une étape de déprotection, d'oxydation ou de réduction.

Dans le cas où **P** est identique à **R**, les conditions de déprotection du groupement **P** provoquent également la déprotection de l'amine portant le groupement **R** et le composé de formule **I** est directement obtenu à la fin de l'étape (b'). Auquel cas, l'étape

(c') n'est pas nécessaire. Ce cas de figure se présente notamment lorsque **R₃** est égal à OH, notamment lorsque **P** et **R** sont tous deux un benzyle, retiré par exemple par une réaction d'hydrogénation.

### Etape (b):

L'étape (b) correspond à une étape de déprotection de l'amine portant le groupement **OR** pour conduire au composé de formule **I,** dans lequel ladite amine porte un groupement OH.

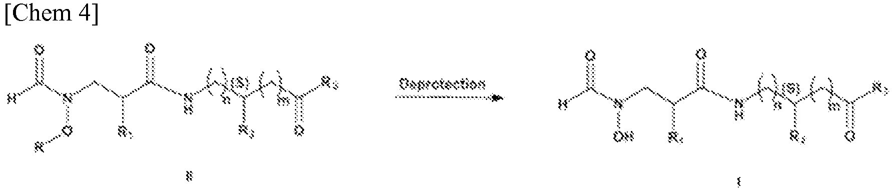

Le composé **I** est obtenu sous forme d'au moins 2 diastéréoisomères. Les diastéréoisomères sont séparés selon des méthodes bien connues de l'homme du métier, typiquement par HPLC préparative ou semi-préparative, pour obtenir un composé **I** sous forme diastéréoisomériquement pur dans lequel les carbones portant **R₁, R₂** et éventuellement **R₈** ou **R₉** (selon la définition de **R₃**) sont résolus et de configuration absolue respective optimisant les propriétés des composés de l'invention. En particulier, le carbone portant **R₁** est de configuration absolue (R), et le carbone portant **R₂** est de configuration absolue (S). Lorsqu'il est présent, le carbone portant **R₈** ou **R₉** est de configuration absolue (R) ou (S).

Ainsi, de manière préférentielle, le composé de formule **I** peut correspondre au diasteréoisomère suivant: 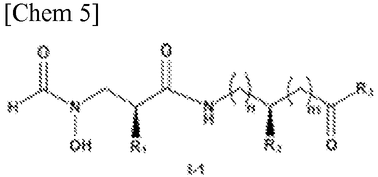 dans lequel **R₃** représente préférablement OH.

### Synthèse énantiosélective

Les composés de formule (II) peuvent également être synthétisés de manière énantiosélective à l'aide de la copule d'Oppolzer (Heravi M M & Zadsirjan V (2014), Tetrahedron : Asymmetry, 1061-1090) selon les étapes suivantes :
1° Le dérivé d'acide acrylique **V** réagit avec du chlorure de thionyle SOCl₂ pour conduire au chlorure d'acide **X** : 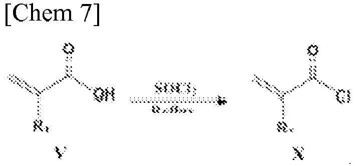
2° Le chlorure d'acide **X** est ensuite couplé à la copule d'Oppolzer ((1R)-(+)-2,10-Camphorsultam) en présence d'une base forte pour donner le composé **XI** : 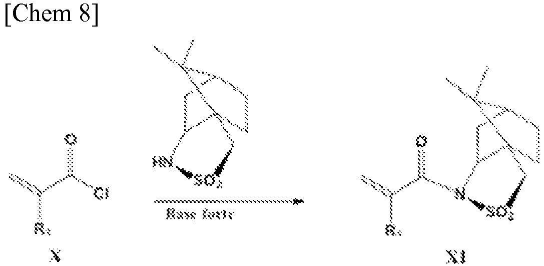
3° L'addition de Michael stéréosélective de l'hydroxylamine **VI** sur le composé **XI** permet d'obtenir de manière optiquement pure le composé **XII** : 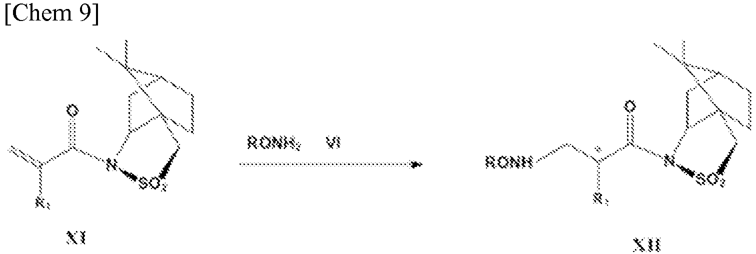
4° La copule chirale est clivée par une base forte pour donner le composé optiquement pur **XIII-1** *(*Naeslund C et al. (2005), Tetrahedron, 61, 1181-1186) : 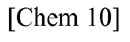 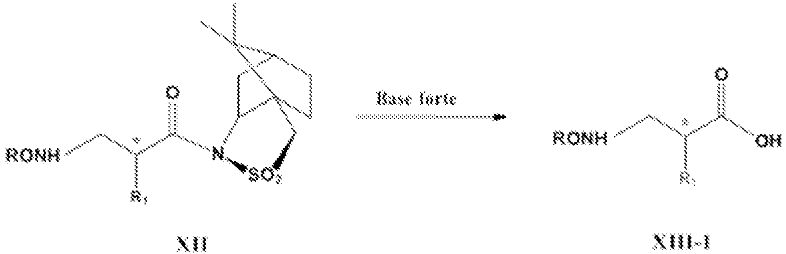
5° Les étapes de synthèse conduisant aux composés **III-1** et **II-1** sont identiques à celles utilisées pour la synthèse des produits racémiques, décrite précédemment.

### EXEMPLES

L'invention sera encore illustrée sans n'être aucunement limitée par les exemples ci-après.

### Liste des abréviations :

Ac₂O: Anhydride acétique
AcOEt: Acétate d'éthyle
Bn : Benzyle
DIEA : Diisopropyléthylamine
DMF : Diméthyl formamide
DMSO : Diméthyl sulfoxyde
HPLC : Chromatographie liquide haute performance
Ph : Phényle
Rdt : Rendement
RMN : Résonnance magnétique nucléaire
Rt : Temps de rétention
TFA: Acide trifluoroacétique

### Synthèse des composés de l'invention

Les acides acryliques **V** sont commerciaux ou bien encore synthétisés comme décrits dans Organic Synthèses, (1955) coll. Vol. 3, p.377*; (*1945) vol.25, p.42*.*

La configuration absolue du carbone portant R₁ a été définie après séparation des 2 diastéréoisomères **Ia** et analyse par RMN du proton du déplacement chimique du groupement CH₃, en appliquant la règle établie par Fournie-Zaluski et al dans J Med Chem (1986), 29, 751-753*.*

La configuration absolue attribuée au composé **Ia-1** est donc (2R, 3S) et celle du composé **Ia-2** est (2S, 3S). Les configurations absolues des analogues ont été fixées par analogie de structure stéréochimique du bon diastéréoisomère ayant la meilleure affinité enzymatique.

### Etape 1 : synthèse des acides 2-alkyl-3-(benzylamino) propanoïque VIIa pour lesquels R = Bn

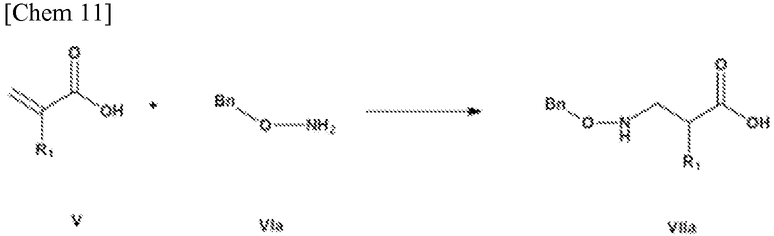

La benzylhydroxylamine **VIa** (4 eq) réagit avec l'acide acrylique **V** à 50°C pendant 8 h. Le mélange est repris dans l'AcOEt. L'excès de benzylhydroxylamine est éliminé par lavage avec HCl 1N. La phase organique est lavée par une solution saturée de NaCl, séchée sur Na₂SO₄ et concentrée sous pression réduite. L'huile est triturée dans l'éther pour donner le composé solide **VIIa.**

### VIIa-1 acide 2-benzyl-3-(benzyloxyamino) propanoïque

R**₁** = CH₂Ph: solide, (Rdt: 80,9 %)
RMN (DMSO + TFA, 400 MHz): 2,7-3,3 (4H, m); 3,51 (1H, q); 4,95 (2H, s); 7,1-7,4 (10H, m)
HPLC Kromasil C18, 4,6 x 250 mm, CH₃CN/H₂O (0,05% TFA) 6/4 Rt= 7,3 min

### VIIa-2 acide 3-(benzyloxyamino)-2-(biphenyl-4-ylmethyl) propanoïque

R₁ = CH₂Ph(4-Ph): solide, (Rdt: 87,7%)
RMN (DMSO + TFA, 400 MHz): 2,8-3,1 (3H, m); 3,25-3,35 (1H, q); 3,55 (1H, q); 4,95 (2H, s); 7,1-7,6 (14H, m)
HPLC Kromasil C18, 4,6 x 250 mm, CH₃CN/H₂O (0,05% TFA) 7/3 Rt= 8,7 min

### Etape 2 : synthèse des acides 2-alkyl-3-(N-(benzyloxy)formamido) propanoïque (IIIa, R=Bn)

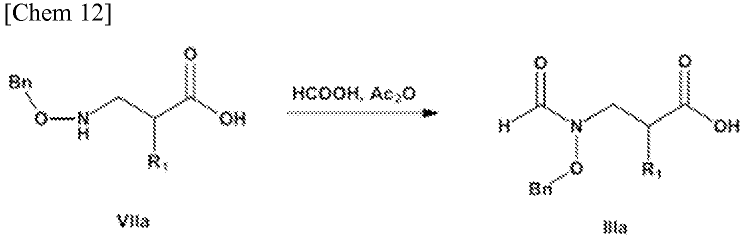

L'acide **VIIa** est solubilisé à 0°C dans l'acide formique. L'anhydride acétique est ajouté à 0°C et le mélange est agité pendant 3h à 0°C. Le mélange est repris dans l'éther puis le mélange est évaporé à sec pour donner l'acide **IIIa** qui est utilisé tel quel pour l'étape suivante.

### IIIa-1 acide 2-benzyl-3-(N-(benzyloxy)formamido) propanoïque

R**₁** = CH₂Ph: huile, (Rdt: 100%)
RMN (DMSO + TFA, 400 MHz): 2,6-3,0 (4H, m); 3,51 (1H, q); 4,80 (2H, s); 7,1-7,4 (10H, m); 7,85 (0,5H, s), 8,15 (0,5H, s) (C**H**O) cis/trans
HPLC Kromasil C18, 4,6 x 250 mm, CH₃CN/H₂O (0,05% TFA) 6/4 Rt= 7,2 min

### IIIa-2 acide 3-(N-(benzyloxy)formamido)-2-(biphenyl-4-ylmethyl) propanoïque

R**₁** = CH₂Ph(4-Ph): huile, (Rdt: 100%)
RMN (DMSO + TFA, 400 MHz): 2,8-3,1 (3H, m); 3,25-3,35 (1H, q); 3,55 (1H, q); 4,95 (2H, s); 7,1-7,6 (14H, m); 7,85 (0,5H, s), 8,15 (0,5H, s) (C**H**O) cis/trans HPLC Kromasil C18, 4,6 x 250 mm, CH₃CN/H₂O (0,05% TFA) 7/3 Rt= 8,7 min

### Etape 3 : couplage acide aminé, synthèse des composés IIa

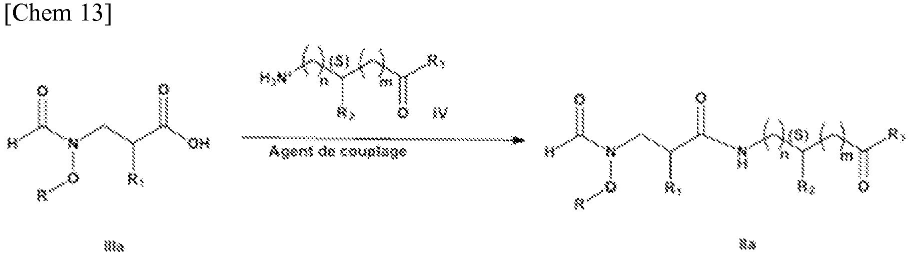

L'acide **IIIa** et le sel d'acide aminé, ester benzylique (1,2 eq) **IV** sont solubilisés dans le DMF à 0°C. TBTU (1,2 eq) et le DIEA (3 eq) sont ajoutés et le mélange est agité pendant 15 min à température ambiante. Le DMF est évaporé sous pression réduite et le mélange est repris dans l'AcOEt. La phase organique est lavée par une solution aqueuse à 10 % d'acide citrique, une solution saturée et est séchée sur Na₂SO₄.

Le mélange brut est purifié par HPLC semi-préparative sur colonne Kromasil C18, 21,2 x 250 mm avec CH₃CN/H₂O (0,05% TFA) 65/35 comme système d'élution pour donner le composé **IIa.**

### IIa-1 acide (2S)-benzyl 2-(2-benzyl-3-(N-(benzyloxy)formamido)propanamido) propanoïque

R**₁** = CH₂Ph, R₂ = (S)-CH₃, n=m=0: solide, (Rdt: 58,2%)
RMN (DMSO + TFA, 400 MHz): 1,0-1,3 (3H, m); 2,5-3,1 (3H, m); 3,15-3,60 (1H, m), 3,60 (1H, qt); 4,25 (1H, q); 4,6-4,85 (2H, m); 5,15 (2H, s); 7,0-7,4 (10H, m); 7,7-8,15 (1H, m); 8,3-8,6 (1H, m)
HPLC Kromasil C18, 4,6 × 250 mm, CH₃CN/H₂O (0,05% TFA) 7/3, Rt= 15,1 et 16,6 min
ESI (+): [M + Na]⁺ = 497,35; [(M-CH₂Ph) + Na]⁺ = 406,24

### IIa-2 acide (2S)-benzyl 2-(2-benzyl-3-(N-(benzyloxy)formamido)propanamido)-3-phenyl propanoïque

R**₁** = CH₂Ph, R₂ = (S)-CH₂Ph, n=m=0: solide, (Rdt: 64,5%)
HPLC Kromasil C18, 4,6 x 250 mm, CH₃CN/H₂O (0,05% TFA) 6/4, Rt= 16,9 et 17,9 min
ESI (+): [M + Na]⁺ = 573,25; [(M-CH₂Ph) + Na]⁺ = 482,14

### IIa-3 acide (3S)-phenyl 3-(2-benzyl-3-(N-(benzyloxy)formamido)propanamido)-4-phenyl butanoïque

R**₁** = CH₂Ph, R₂ = (S)-CH₂Ph, n=0; m=1: solide, (Rdt: 66,5%)
HPLC Kromasil C18, 4,6 x 250 mm, CH₃CN/H₂O (0,05% TFA) 6/4, Rt= 15,2 et 16,1 min
ESI (+): [M + Na]⁺ = 587,26; [(M-CH₂Ph) + Na]⁺ = 496,14

### IIa-4 acide (2S)-benzyl 2-(3-(N-(benzyloxy)formamido)-2-(biphenyl-4-ylmethyl)propanamido) propanoïque

R₁ = CH₂Ph(4-Ph), R₂ = (S)-CH₃, n=m=0: solide, (Rdt: 59,2%)
HPLC Kromasil C18, 4,6 x 250 mm, CH₃CN/H₂O (0.05% TFA) 7/3, Rt= 16,2 et 17,2 min
ESI (+): [M + Na]⁺ = 497,26; [(M-CH₂Ph) + Na]⁺ = 406,14

### IIa-5 acide (2S)-phenyl 2-benzyl-3-(2-benzyl-3-(N-(benzyloxy)formamido) propanamido) propanoïque

R**₁** = CH₂Ph, R₂ = (S)-CH₂Ph, n=1, m=0: huile incolore, (Rdt: 31,0%)
HPLC Kromasil C18, 4,6 x 250 mm, CH₃CN/H₂O (0,05% TFA) 7/3, Rt= 10,6 et 11,3 min
ESI (+): [M + Na]⁺ = 587,26; [(M-CH₂Ph) + Na]⁺ = 496,14

La (S)-Alanine ester benzylique (R₂ = (S)-CH₃, n= m=0), la (S)-Phenyl alanine ester benzylique (R₂ = (S)-CH₂Ph, n= m=0) sont commerciaux.

La (S)-béta-Homophenylalanine ester benzylique (R₂ = (S)-CH₂Ph, n=0, m=1), la (R)-béta-2-Homophenylalanine ester benzylique (R₂ = (S)-CH₂Ph, n=1, m=0) sont fabriquées à partir respectivement de Boc (S)-béta-Homophenylalanine et Boc (R)-béta-2-Homophénylalanine commerciaux par estérification en ester benzylique et déprotection du Boc comme décrit dans la littérature et connu de l'homme de l'art (Hassner A et Alexanian V (1978), Tetrahedron Lett, 19, 4475*;* Ripka AS et al. (1998), Bioorg Med Chem Lett, 8, 357*).*

### Etape 4 : Déprotection du composé IIa et séparation des diastéréoisomères I pour lesquels R₃ = OH

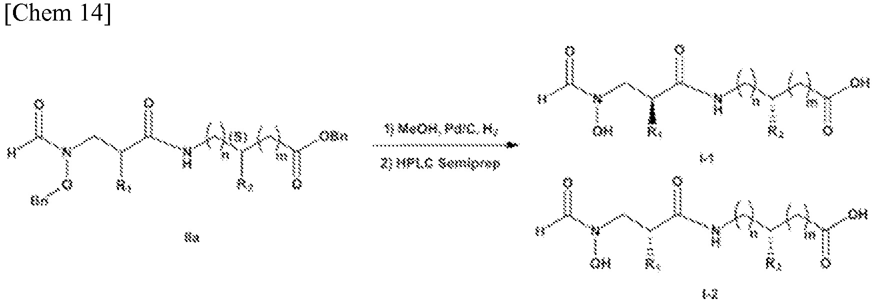

Le composé **IIa** est solubilisé dans MeOH. Pd/C est ajouté et le mélange est placé sous atmosphère d'hydrogène pendant 2h à température ambiante. La conversion est suivie par HPLC. Le Pd/C est filtré sur célite. Le solvant est évaporé sous pression réduite pour donner un mélange qui est purifié par HPLC semi-préparative, sur colonne Kromasil C18, 21,2 x 250 mm avec CH₃CN/H₂O (0,05% TFA) 35/65 comme système d'élution pour séparer les 2 diastéréoisomères **I**.

### Ia-1 acide (S)-2-((R)-2-benzyl-3-(N-hydroxyformamido)propanamido) propanoïque

R₁ = (R)-CH₂Ph, R₂ = (S)-CH₃, n=m=0: solide, (Rdt: 31,6 %)
RMN (DMSO + TFA, 400 MHz): 1,15 (3H, t); 2,5-3,2 (4H, m); 3,35-3,70 (1H, m); 4,15 (1H, t); 7,1-7,3 (5H, m); 7,7-8,15 (1H, m) et 8,1-8,3 (1H, m) (C**H**O, isomérie cis/trans) HPLC Kromasil C18, 4,6 x 250 mm, CH₃CN/H₂O (0,05% TFA) 3/7, Rt= 6,2 min

### Ia-2 acide (S)-2-((S)-2-benzyl-3-(N-hydroxyformamido)propanamido) propanoïque

R₁ = (S)-CH₂Ph, R₂ = (S)-CH₃, n=m=0: solide, (Rdt: 23,4 %)
RMN (DMSO + TFA, 400 MHz): 1,0 (3H, t); 2,5-2,75 (2H, m); 2,95 (1H, m); 3,35-3,70 (2H, m); 4,05 (1H, qt); 7,1-7,3 (5H, m); 7,8-8,25 (1H, m) et 8,05-8,2 (1H, m) (C**H**O, isomérie cis/trans)
HPLC Kromasil C18, 4,6 × 250 mm, CH₃CN/H₂O (0,05% TFA) 3/7, Rt= 8,5 min

### Ib-1 acide (S)-2-((R)-2-benzyl-3-(N-hydroxyformamido )propanamido )-3-phenylpropanoïque

R₁ = (R)-CH₂Ph, R₂ = (S)-CH₂Ph, n=m=0: solide, (Rdt: 23,0 %)
RMN (DMSO + TFA, 400 MHz): 2,6-3,1 (5H, m); 3,15-3,40 (2H, m), 3,55 (1H, q); 4,40 (1H, m); 6,9-7,3 (10H, m); 7,7-8,15 (1H, m) et 8,20-8,35 (1H, m) (C**H**O, isomérie cis/trans)
HPLC Kromasil C18, 4,6 x 250 mm, CH₃CN/H₂O (0,05% TFA) 35/65, Rt= 12,2 min

### Ib-2 acide (S)-2-((S)-2-benzyl-3-(N-hydroxyformamido)propanamido)-3-phenylpropanoïque

R**₁** = (S)-CH₂Ph, R₂ = (S)-CH₂Ph, n=m=0: solide, (Rdt: 29,0 %)
RMN (DMSO + TFA, 400 MHz): 2,6-3,1 (5H, m); 3,15-3,40 (2H, m), 3,55 (1H, q); 4,40 (1H, m); 6,9-7,3 (10H, m); 7,7-8,15 (1H, m) et 8,10-8,35 (1H, m) (C**H**O, isomérie cis/trans)
HPLC Kromasil C18, 4,6 x 250 mm, CH₃CN/H₂O (0,05% TFA) 35/65, Rt= 20,5 min

### Ic-1 acide (S)-3-((R)-2-benzyl-3-(N-hydroxyformamido)propanamido)-4-phenyl butanoïque

R**₁** = (R)-CH₂Ph, R₂ = (S)-CH₂Ph, n=0, m=1: solide, (Rdt: 35,0 %)
RMN (DMSO + TFA, 400 MHz): 2,15-2,85 (7H, m); 3,15-3,40 (2H, m); 4,25 (1H, m); 6,9-7,3 (10H, m); 7,6-8,3 (2H, m) (C**H**O, isomérie cis/trans)
HPLC Kromasil C18, 4,6 × 250 mm, CH₃CN/H₂O (0,05% TFA) 35/65, Rt= 9,95 min

### Ic-2 acide (S)-3-((R)-2-benzyl-3-(N-hydroxyformamido)propanamido)-4-phenyl butanoïque

R**₁** = (S)-CH₂Ph, R₂ = (S)-CH₂Ph, n=0, m=1: solide, (Rdt: 40,0 %)
RMN (DMSO + TFA, 400 MHz): 2,6-3,1 (7H, m); 3,2-3,60 (2H, m); 4,15 (1H, m); 6,9-7,2 (10H, m); 7,7- 8,4(2H, m) (C**H**O, isomérie cis/trans)
HPLC Kromasil C18, 4,6 × 250 mm, CH₃CN/H₂O (0,05% TFA) 35/65, Rt= 16,7 min

### Id-1 acide (S)-2-((R)-3-(biphenyl-4-yl)-2-(N-hydroxyformamido )propanamido) propanoïque

R₁ = (R)-CH₂Ph(4-Ph), R₂ = (S)-CH₃, n=m=0: solide, (Rdt: 21,9 %)
RMN (DMSO + TFA, 400 MHz): 1,15 (3H, t); 2,5-3,2 (4H, m); 3,40-3,70 (1H, m); 4,15 (1H, t); 7,1-7,5 (9H, m); 7,7- 8,4(2H, m) (C**H**O, isomérie cis/trans)
HPLC Kromasil C18, 4,6 × 250 mm, CH₃CN/H₂O (0,05% TFA) 4/6, Rt= 9,6 min

### Id-2 (S)-2-((S)-3-(biphenyl-4-yl)-2-(N-hydroxyformamido)propanamido)propanoïque

R₁ = (S)-CH₂Ph(4-Ph), R₂ = (S)-CH₃, n=m=0: solide, (Rdt: 29,2 %)
RMN (DMSO + TFA, 400 MHz): 1,0 (3H, t); 2,5-2,75 (2H, m); 3,0 (1H, m); 3,30-3,70 (2H, m); 4,05 (1H, qt); 7,1-7,55 (9H, m); 7,7- 8,4(2H, m) (C**H**O, isomérie cis/trans) HPLC Kromasil C18, 4,6 × 250 mm, CH₃CN/H₂O (0,05% TFA) 4/6, Rt= 12,6 min

### Ie-1 acide (S)-2-benzyl-3-((R)-2-benzyl-3-(N-hydroxyformamido)propanamido) propanoïque

R₁ = (R)-CH₂Ph, R₂ = (S)-CH₂Ph, n=1, m=0: solide, (Rdt: 8,7 %)

RMN (DMSO + TFA, 400 MHz): 2,15-2,85 (9H, m); 3,15-3,40 (2H, m); 7,0-7,4 (10H, m); 7,7-8,2 (2H, m) (C**H**O, isomérie cis/trans)

HPLC Kromasil C18, 4,6 × 250 mm, CH₃CN/H₂O (0,05% TFA) 30/70, Rt= 17,6 min

### Ie-2 acide (S)-2-benzyl-3-((S)-2-benzyl-3-(N-hydroxyformamido)propanamido) propanoïque

R₁ = (S)-CH₂Ph, R₂ = (S)-CH₂Ph, n=1, m=0: solide, (Rdt: 9,6 %)
RMN (DMSO + TFA, 400 MHz): 2,15-2,85 (9H, m); 3,15-3,40 (2H, m); 7,05-7,35 (10H, m); 7,9-8,35 (2H, m) (C**H**O, isomérie cis/trans)
HPLC Kromasil C18, 4,6 × 250 mm, CH₃CN/H₂O (0,05% TFA) 30/70, Rt= 20,0 min

### Synthèse chirale du composé XIIIa-1

### Etape 1 : synthèse du chlorure de l'acide 2-benzylacrylique Xa

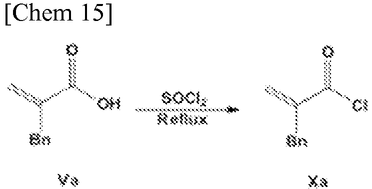

L'acide 2-benzylacrylique **Va** (2g, 12,3 mmol) est porté à reflux dans 14 mL de chlorure de thionyle pendant 3h. L'excès de SOCl₂ est évaporé sous pression réduite. Une huile jaune claire est obtenue de manière quantitative et est utilisé telle quelle pour l'étape suivante.

HPLC Kromasil C18, 4,6 x 250 mm, CH₃CN/H₂O (0,05% TFA) 50/50, Rt= 3,77 min

### Etape 2 : Synthèse de l'adduit acide 2-benzylacrylique-(1R)-(+)-2,10-Camphorsultam Xia

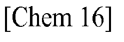

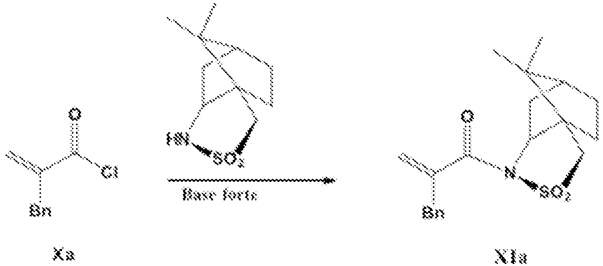

(1R)-(+)-2,10-Camphorsultam (19,7g, 90,0 mmol) est mis en solution dans 200 mL de THF. Le mélange est refroidi à 0°C et NaH 60% dans l'huile (4.4 g, 110 mmol, 1,2 equiv) est ajouté. Le mélange est agité 30 min à 0°C avec formation d'un sel. Le chlorure d'acide **Xa** (19,98 g, 110 mmol, 1,2 equiv) en solution dans 100 mL de THF est ajouté et le mélange est agité à température ambiante 48h. Le THF est évaporé à sec et le mélange est repris dans AcOEt. La phase organique est lavée par HCl 1N, NaHCO₃ 10%, H₂O et une solution saturée de NaCl puis elle est séchée sur Na₂SO₄ avec d'être évaporée à sec pour donner un solide blanc, le composé **XIa** (18,3 g, Rdt : 56%), après recristallisation dans l'éther.

RMN (CDCl₃, 400 MHz): 0,8-2,0 (12H, m); 2,55 (1H, q); 3,05(1H, d); 3,40 (1H, dd); 3,55 (1H, dd); 3,95 (1H, t); 5,30 (1H, s); 5,80 (1H, s); 7,10-7,40 (5H, m)

HPLC Kromasil C18, 4,6 x 250 mm, CH₃CN/H₂O (0,1% TFA) 70/30, Rt= 13,7 min

### Etape 3 : Synthèse de l'adduit acide 2-benzyl-3-(benzyloxyamino) propanoïque -(1R)-(+)-2,10-Camphorsultam XIIa

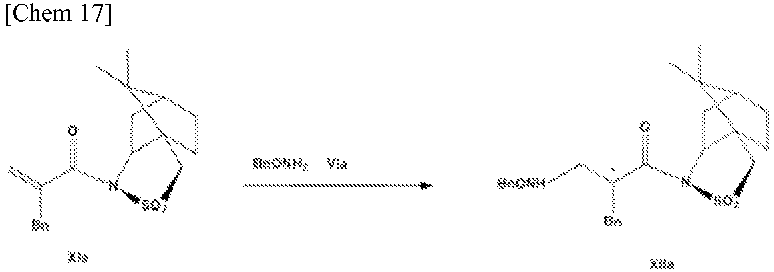

Le composé **XIa** (1,05 g, 2.9 mmol) est mis en solution dans 10 mL de CH₂Cl₂. La benzylhydroxylamine **VIa** (864 mg, 7 mmol, 2,4 equiv) est ajoutée et le mélange est porté à reflux pendant 24h. Le mélange est dilué par CH₂Cl₂ et par de l'eau. La phase organique est lavée par HCl 1N, H₂O, une solution saturée de NaCl, séchée sur Na₂SO₄ puis évaporée à sec pour donner 1,45 g d'une huile incolore.

Après cristallisation dans l'EtOH 790 mg d'un solide blanc (**XIIa**) sont obtenus (Rdt : 56%).

RMN (CDCl₃, 400 MHz): 0,8 (3H, s); 1,0 (3H, s); 1,1-1,9 (7H, m); 2,55 (1H, q); 2,95(1H, dd); 3,05(1H, dd); 3,15 (1H, t); 3,35 (3H, m); 3,60 (4H, m); 3,80 (1H, t); 4,45 (2H, s); 7,10-7,40 (10H, m)

HPLC Symmetry C18, 4,6 x 250 mm, CH₃CN/H₂O (0,1% TFA) 60/40, Rt= 15,0 min

### Etape 4 : Hydrolyse de la copule chirale : synthèse de l'acide 2-benzyl-3-(benzyloxyamino) propanoïque XIIIa-1

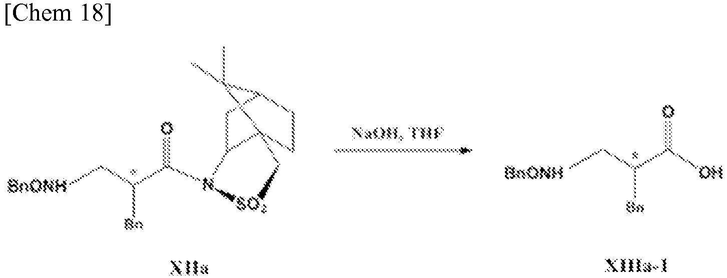

Le composé **XIIa** (700 mg, 1,45 mmol) est mis en solution dans 30 mL de THF. 6 mL de LiOH 1N sont ajoutés et le mélange est agité à 55°C pendant 48h. Le mélange est acidifié jusqu'à pH 1-2 par HCl 1N. Le THF est évaporé à sec et le mélange est repris dans l'AcOEt. La phase organique est lavée par H₂O, une solution saturée de NaCl, séchée sur Na₂SO₄ puis évaporée à sec pour donner 400 mg d'une huile légèrement jaune. Le produit est purifié sur colonne de silice avec CH₂Cl₂/MeOH 9/1 comme système d'élution pour donner 174 mg du composé **XIIIa-1** (Rdt : 44,0%) en tant qu'huile qui cristallise.

RMN (CDCl₃, 400 MHz): 2,75 (1H, m); 2,85-3,10 (1H, q); 4,65 (2H, s); 7,1-7,4 (10H, m)

HPLC Kromasil C18, 4,6 x 250 mm, CH₃CN/H₂O (0,1% TFA) 4/6 Rt= 12,3 min

### Etape 5 : synthèse de l'acide (2S)-benzyl 2-(2-benzyl-3-(N-(benzyloxy)formamido)propanamido) propanoïque IIIa-1

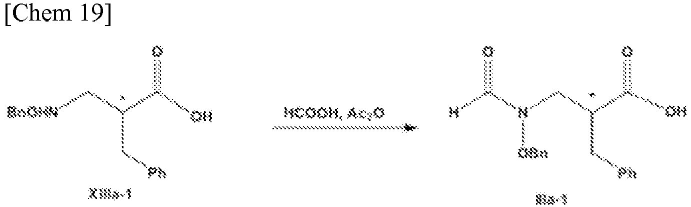

L'acide **XIIIa-1** (12 mg, 0,042 mmol) est solubilisé à 0°C dans l'acide formique (1mL), L'anhydride acétique (0,5 mL) est ajouté à 0°C et le mélange est agité pendant 4h à 0°C. Le mélange est repris dans le dichlorométhane et l'eau. La phase organique est lavée par une solution saturée de NaCl, séchée sur Na₂SO₄ et est évaporée à sec pour donner l'acide **IIIa-1** (13 mg, Rdt : 100%) sous forme d'huile incolore qui est utilisée tel quelle pour l'étape suivante.

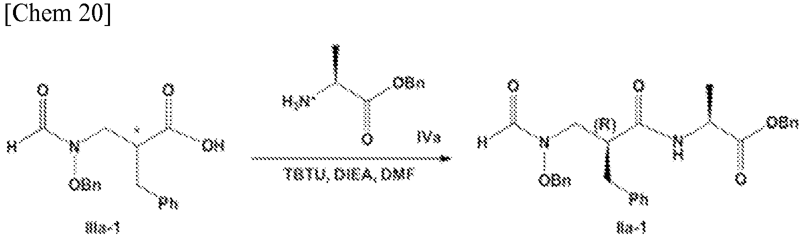

L'acide **IIIa-1** (13 mg, 0,042 mmol), précédemment décrit et le sel de l'ester benzylique de la (S)-Alanine (11 mg, 0,054 mmol, 1,3 eq) sont solubilisés dans 1 mL de DMF à 0°C, TBTU (12 mg, 0,052 mmol, 1,3 eq) et le DIEA (22 µL, 0,126 mmol, 3 eq) sont ajoutés et le mélange est agité pendant 15 min à température ambiante. Le DMF est évaporé sous pression réduite et le mélange est repris dans l'AcOEt. La phase organique est lavée par une solution aqueuse à 10 % d'acide citrique, une solution saturée et est séchée sur Na₂SO₄.

Le mélange brut est purifié par HPLC semi-préparative sur colonne Kromasil C18, 21,2*250 mm avec CH₃CN/H₂O (0,1% TFA) 65/35 comme système d'élution pour donner 3,8 mg du composé **IIa-1** (Rdt = 19,0%).

RMN (DMSO + TFA, 400 MHz): 1,3 (3H, t); 2,8-3,8 (5H, m); 4,25 (1H, q); 4,65-4,85 (2H, m); 5,15 (2H, s); 7,0-7,4 (10H, m); 7,7 et 8,15 (1H, m) C**H**O (isomérie cis/trans); 8,45-8,65 (1H, NH, m) (isomérie cis/trans)

HPLC Symmetry C18, 4,6 x 250 mm, CH₃CN/H₂O (0,1% TFA) 6/4, Rt= 11,94 (96,0% dia 2R, 3S) et 11,69 min (4,0 % dia 2S, 3S)

La configuration absolue du carbone portant le groupement benzyle est définie par analyse RMN, en appliquant la règle établie par Fournie-Zaluski et al dans J Med Chem (1986), 29, 751-753*.*

La configuration absolue attribuée au composé **IIa-1** est donc (2R, 3S) et l'excès énantiomérique obtenu lors de l'addition de Michael en présence de la copule chirale est donc de 96 %. Cette synthèse chirale peut également s'appliquer aux autres analogues.

### Mesure des pouvoirs inhibiteurs

Les inhibiteurs revendiqués ont été testés sur différentes peptidases à Zn²⁺ représentatives de cette famille d'enzymes, susceptibles de les inhiber, telles que la néprilysine de type 1 (E.C.3.4.24.11, NEP-1), l'aminopeptidase neutre (E.C.3.4.11.2, APN) et la LTA4H.

Les dosages sont effectués en plaque 96 puits en présence de substrats fluorogéniques spécifiques de chaque enzyme.

De manière générale, les inhibiteurs sont pré-incubés, en concentrations croissantes, avec l'enzyme pendant 10 min, à 37°C. Le substrat est alors ajouté et le mélange est incubé pendant 30 à 60 min à 37°C. La réaction est arrêtée à 4°C et la lecture de la fluorescence émise est faite dans un lecteur de plaque Berthold Twinkle LS970B. On réalise ensuite une courbe d'inhibition en fonction de la concentration en inhibiteur à l'aide du logiciel GraphPad, puis on détermine le Ki à partir de la formule de Cheng Prusoff : Ki=IC₅₀/(1+(S/Km)).

### Dosage de la néprilysine (NEP)

La néprilysine, purifiée à partir de rein de lapin (Aubry M et al. (1987), Biochem Cell Biol 65, 398-404), est utilisée à 200 ng/mL final dans le tampon Tris 50 mM pH 7,4. Le substrat, Dansyl-Gly-(NO₂)Phe-ß-Ala (GoudreauN et al. (1994), Anal Biochem, 219, 87-95) (Km=37 µM), est dissous dans l'éthanol et est utilisé à 20 µM final. Des concentrations croissantes (de 10⁻¹⁰ à 10⁻³ M) d'inhibiteurs sont pré-incubées pendant 15 min à 37°C avec la NEP dans le tampon Tris 50 mM, pH 7,4. Le substrat est ensuite ajouté et l'incubation est poursuivie pendant 60 min. La réaction est arrêtée en mettant la plaque dans la glace pendant 10 min. La lecture de la fluorescence émise est effectuée dans un fluorimètre à λex= 355 nm, λem= 535 nm.

### Dosage de l'aminopeptidase neutre (APN)

La mesure de l'inhibition de l'aminopeptidase N (APN) est effectuée par utilisation du substrat L-Ala↓ß-NA (50 µM, Sigma Aldrich). Les pouvoirs inhibiteurs sont déterminés en utilisant de l'enzyme humaine recombinante (rh) (50 ng/mL ; R&D System). Des concentrations croissantes (de 10⁻¹⁰ à 10⁻³ M) d'inhibiteurs sont pré-incubées pendant 30 min à 37°C avec l'APN-rh dans le tampon Tris 50 mM, pH 7,4. Le substrat est ensuite ajouté et l'incubation est poursuivie pendant 30 min à 37°C. La réaction est arrêtée en mettant la plaque dans la glace pendant 10 min. La lecture de la fluorescence émise est mesurée dans un fluorimètre à λex= 340 nm, λem= 405 nm.

### Dosage de la LTA4 hydrolase

Pour déterminer les valeurs de Ki des différents inhibiteurs vis-à-vis de LTA4H, 0,6 µg / mL d'enzyme recombinante ont été préalablement incubés pendant 30 minutes à 37°C dans du Tris HCl 50 mM, pH 7,4, NaCl 100 mM, avec des concentrations croissantes d'inhibiteur (de 10⁻¹⁰ M à 10⁻⁴ M de concentration finale). Le substrat fluorescent (L)-Ala-β-naphthylamide (1 mM) est ajouté dans un volume final de 100 µL et est incubé à 37°C pendant 15 minutes. Les valeurs de fluorescence sont mesurées sur un Berthold Twinkle LB 970 (λex = 340 nm, λem = 405 nm, énergie de la lampe 10000). Des échantillons contenant 0 % d'hydrolyse ont été obtenus en ajoutant le substrat au tampon et des échantillons présentant une activité relative de 100 % ont été préparés sans inhibiteur. Le pourcentage de clivage a été évalué et comparé à une activité relative de 100 %, et les valeurs de la IC₅₀ ont été déterminées en conséquence. Les valeurs de Ki des inhibiteurs (moyenne d'au moins trois dosages indépendants en double) ont été calculées à l'aide de l'équation Ki = IC₅₀ / (1 + [S] / Km).

Résultats : Pouvoirs inhibiteurs NEP, APN, LTA4H

**H-CO-N(OH)-CH₂-CH(R₁)-CO-NH-(CH₂)ₙCH(R₂)(CH₂)ₘ-CO-R₃** **(1)**

**[Table 1]**

| **Composé** | **R₁** | **R₂** | **n, m** | **R₃** | **NEP (nM)** | **APN (nM)** | **LTA4H (nM)** |
|---|---|---|---|---|---|---|---|
| **Ia-1** | (R)-CH₂Ph | (S)-CH₃ | n=m=0 | OH | 3,3±0,7 | 143±9 | 7,5±0,4 |
| **Ia-2** | (S)-CH₂Ph | (S)-CH₃ | n=m=0 | OH | 17,7±0,9 | >10000 | nd |
| **Ib-1** | (R)-CH₂Ph | (S)-CH₂Ph | n=m=0 | OH | 0,5±0,1 | 24,0±0,4 | 26±2 |
| **Ib-2** | (S)-CH₂Ph | (S)-CH₂Ph | n=m=0 | OH | 23,1±1,1 | >10000 | nd |
| **Ic-1** | (R)-CH₂Ph | (S)-CH₂Ph | n=0, m=1 | OH | 6,3±0,3 | 12,0±1,7 | 443±75 |
| **Ic-2** | (S)-CH₂Ph | (S)-CH₂Ph | n=0, m=1 | OH | 54,6±2,4 | 624±20 | >10000 |
| **Id-1** | (R)-CH₂Ph-4-Ph | (S)-CH₃ | n=m=0 | OH | 0,08±0,01 | 86±8 | 6,1±0,7 |
| **Id-2** | (S)-CH₂Ph-4-Ph | (S)-CH₃ | n=m=0 | OH | 44,1±2,2 | 817±80 | >10000 |
| **Ie-1** | (S)-CH₂Ph | (S)-CH₂Ph | n=1, m=0 | OH | 30,2±2,8 | 1750±20 | nd |
| **Ie-2** | (R)-CH₂Ph | (S)-CH₂Ph | n=1, m=0 | OH | 2,6±0,1 | 35,3±3,8 | nd |

| | | | | | | | |
|---|---|---|---|---|---|---|---|
| nd : non détermine | | | | | | | |

Les composés Ia-2, Ib-2, Ic-2, Id-2 et Ie-2 sont des exemples comparatifs.

### Test pharmacologique

La figure 1 montre que l'association intraveineuse de THC et du composé **Ib-1** à des doses inactives dans ce test induit un effet analgésique synergique considérable, 10 min après injection, dans le test de la plaque chaude sur les souris OF1 males.

## Revendications

1. Composé de formule (I) suivante :
**H-CO-N(OH)-CH₂-CH(R₁)-CO-NH-(CH₂)ₙ-CH(R₂)-(CH₂)ₘ-CO-R₃** **(I)**
dans lequel :
R**₁** représente un groupement hydrocarboné linéaire ou ramifié comprenant de 1 à 6 atomes de carbone substitué par un ou plusieurs groupements choisis parmi :
- un aryle, lui-même non substitué ou substitué par un ou plusieurs groupements choisis parmi les halogènes tels que le fluor et le brome, un groupement phényle, un groupement benzyle, un groupement OR₄, R₄ étant choisi parmi un hydrogène et un groupement alkyle linéaire ou ramifié comprenant de 1 à 4 atomes de carbone, et leurs combinaisons,
- un hétéroaryle à 5 ou 6 chaînons comprenant 1 ou 2 hétéroatomes, chaque hétéroatome étant choisi parmi l'oxygène, l'azote et le soufre, et
- un cyclohétéroalkyle à 5 ou 6 chaînons comprenant 1 ou 2 hétéroatomes, chaque hétéroatome étant choisi parmi l'oxygène, l'azote et le soufre,
**R₂** représente:
- un groupement hydrocarboné linéaire ou ramifié comprenant de 1 à 6 atomes de carbone, non substitué ou substitué par un ou plusieurs groupements choisis parmi :
• un groupement choisi parmi OR₅, SR₅ et S(O)R₅, R₅ étant choisi parmi un hydrogène et un groupement alkyle linéaire ou ramifié comprenant de 1 à 4 atomes de carbone,
• un groupement CO₂R₆, R₆ étant choisi parmi un hydrogène, un groupement alkyle linéaire ou ramifié comprenant de 2 à 4 atomes de carbone et un groupement benzyle,
• un aryle, lui-même non substitué ou substitué par un ou plusieurs groupements choisis parmi les halogènes, tels que le fluor et le brome, un groupement OR₅, R₅ ayant la même définition que ci-dessus, et leurs combinaisons,
• un hétéroaryle à 5 ou 6 chaînons comprenant 1 ou 2 hétéroatomes, chaque hétéroatome étant choisi parmi l'oxygène, l'azote et le soufre,
• un cycloalkyle à 5 ou 6 chaînons, et
• un cyclohétéroalkyle à 5 ou 6 chaînons comprenant 1 ou 2 hétéroatomes, chaque hétéroatome étant choisi parmi l'oxygène, l'azote et le soufre,
- un aryle, non substitué ou substitué par un ou plusieurs groupements choisis parmi les halogènes, tels que le fluor et le brome, un groupement OR₅, R₅ ayant la même définition que ci-dessus, et leurs combinaisons, ou
- un hétéroaryle comprenant 1 ou plusieurs hétéroatomes, de préférence 1 ou 2, chaque hétéroatome étant choisi parmi l'oxygène, l'azote et le soufre, non substitué ou substitué par un ou plusieurs groupements choisis parmi les halogènes, tels que le fluor et le brome, un groupement OR₅, R₅ ayant la même définition que ci-dessus, et leurs combinaisons,
**R₃** représente :
- un groupement OR₇, R₇ étant choisi parmi :
• un hydrogène,
• un groupement alkyle linéaire ou ramifié comprenant de 2 à 6 atomes de carbone,un groupement benzyle, et
• un groupement CHR₈-COOR₉, CHR₈-O-C(=O)R₉ ou CHR₈₋O-C(=O)-OR₉ dans lesquels R₈ et R₉ sont, indépendamment l'un de l'autre, choisis parmi un groupement alkyle, un groupement aryle, un groupement arylalkyle, un groupement cycloalkyle, un groupement cyclohétéroalkyle, un groupement hétéroalkyle, un groupement hétéroaryle et un groupement hétéroarylalkyle, et
m et n sont indépendamment l'un de l'autre un nombre entier choisi parmi 0 et 1,
l'atome de carbone portant R**₁** ayant une configuration absolue (R) et l'atome de carbone portant **R₂** ayant une configuration absolue (S),
ainsi que leurs sels et/ou solvates pharmaceutiquement acceptables.

2. Composé selon la revendication 1, dans lequel R**₁** représente un groupement hydrocarboné linéaire ou ramifié comprenant de 1 à 6 atomes de carbone, substitué par un aryle, lui-même non substitué ou substitué par un ou plusieurs groupements choisis parmi les halogènes tel que le fluor et le brome, un groupement phényle, un groupement benzyle, un groupement OR₄, R₄ étant choisi parmi un hydrogène et un groupement alkyle linéaire ou ramifié comprenant de 1 à 4 atomes de carbones, et leurs combinaisons.

3. Composé selon la revendication 1 ou 2, dans lequel **R₂** représente un groupement hydrocarboné linéaire ou ramifié comprenant de 1 à 6 atomes de carbone, non substitué ou substitué par un ou plusieurs groupements choisis parmi :
• un aryle, lui-même non substitué ou substitué par un ou plusieurs groupements choisis parmi les halogènes tels que le fluor et le brome, un groupement OR₅, R₅ ayant la même définition qu'à la revendication 1, et leurs combinaisons,
• un cycloalkyle à 5 ou 6 chaînons, et
• un cyclohétéroalkyle à 5 ou 6 chaînons comprenant 1 ou 2 hétéroatomes, chaque hétéroatome étant choisi parmi l'oxygène, l'azote et le soufre.

4. Composé selon l'une quelconque des revendications 1 à 3, dans lequel **R₃** représente un groupement OR₇, R₇ étant choisi parmi :
• un hydrogène, et
• un groupement alkyle linéaire ou ramifié comprenant de 2 à 6 atomes de carbone.

5. Composé selon l'une quelconque des revendications 1 à 4, dans lequel :
R**₁** représente un groupement hydrocarboné linéaire ou ramifié comprenant de 1 à 6 atomes de carbone, substitué par un aryle, lui-même non substitué ou substitué par un ou plusieurs groupements choisis parmi le fluor, le brome, un groupement phényle, un groupement benzyle, un groupement OR₄, R₄ étant choisi parmi un hydrogène et un groupement alkyle linéaire ou ramifié comprenant de 1 à 4 atomes de carbone, et leurs combinaisons,
**R₂** représente un groupement hydrocarboné linéaire ou ramifié de 1 à 6 atomes de carbone,
non substitué ou substitué, par un ou plusieurs groupements choisis parmi :
• un aryle, lui-même non substitué ou substitué par un ou plusieurs groupements choisis parmi le fluor, le brome, un groupement OR₅, R₅ ayant la même définition qu'à la revendication 1, et leurs combinaisons,
• un hétéroaryle à 5 ou 6 chaînons comprenant 1 ou 2 hétéroatomes, chaque hétéroatome étant choisi parmi l'oxygène, l'azote et le soufre,
• un cycloalkyle à 5 ou 6 chaînons, et
• un cyclohétéroalkyle à 5 ou 6 chaînons comprenant 1 ou 2 hétéroatomes, chaque hétéroatome étant choisi parmi l'oxygène, l'azote et le soufre,
**R₃** représente un groupement OR₇, R₇ étant choisi parmi :
• un hydrogène, et
• un groupement alkyle linéaire ou ramifié comprenant de 2 à 6 atomes de carbone, et
m et n sont indépendamment l'un de l'autre un nombre entier choisis parmi 0 et 1.

6. Composé selon l'une quelconque des revendications 1 à 5, dans lequel R**₁** représente un groupement hydrocarboné, de préférence un groupement alkyle linéaire ou ramifié comprenant de 1 à 6 atomes de carbone, substitué par un aryle, de préférence un groupement phényle, lui-même non substitué ou substitué par un groupement phényle.

7. Composé selon l'une quelconque des revendications 1 à 6, dans lequel **R₂** représente un groupement hydrocarboné, de préférence un groupement alkyle linéaire ou ramifié comprenant de 1 à 6 atomes de carbone, non substitué ou substitué par un aryle.

8. Composé selon l'une quelconque des revendications 1 à 7, dans lequel **R₃** représente un groupement OH.

9. Composé selon l'une quelconque des revendications 1 à 8, dans lequel :
- **R₁** = (R)-CH₂Ph ; **R₂** = (S)-CH₃ ; **R₃** = OH ; n=m=0;
- - **R₁** = (R)-CH₂Ph ; **R₂** = (S)-CH₂Ph ; **R₃** = OH ; n=m=0;
- R**₁** = (R)-CH₂Ph ; **R₂** = (S)-CH₂Ph ; **R₃** = OH ; n=0; m=1;
- R**₁** = (R)-CH₂Ph-4-Ph ; **R₂** = (S)-CH₃ ; **R₃** = OH ; n=m=0;
- R**₁** = (R)-CH₂Ph ; **R₂** = (S)-CH₂Ph ; **R₃** = OH ; n=1; m=0.

10. Composé selon l'une quelconque des revendications 1 à 9 pour son utilisation en tant que médicament.

11. Composé selon l'une quelconque des revendications 1 à 9 pour son utilisation en tant qu'analgésique, anxiolytique ou antidépresseur, notamment pour le traitement de douleurs, aiguës ou chroniques, telles que les douleurs post-opératoires, cancéreuses, traumatologiques, les céphalées, les migraines, les douleurs viscérales, les douleurs neurogéniques, neuropathiques, neuro-inflammatoires, nociceptives ou générales comme la fibromyalgie.

12. Composition pharmaceutique comprenant au moins un composé tel que défini selon l'une des revendications 1 à 9 et au moins un excipient pharmaceutiquement acceptable.

13. Composition pharmaceutique selon la revendication 12 pour son utilisation en tant qu'analgésique, anxiolytique ou antidépresseur, notamment pour le traitement de douleurs, aiguës ou chroniques, telles que les douleurs post-opératoires, cancéreuses, traumatologiques, les douleurs viscérales, les céphalées, les migraines, les douleurs viscérales, les douleurs neurogéniques, neuropathiques, neuro-inflammatoires, nociceptives ou générales comme la fibromyalgie.

14. Composition pharmaceutique selon la revendication 12 ou composition pharmaceutique pour son utilisation selon la revendication 13 comprenant en outre au moins un autre principe actif, notamment un analgésique choisi parmi la morphine et ses dérivés, les endocannabinoïdes et les inhibiteurs de leur métabolisme, les dérivés du GABA tels que la gabapentine ou la prégabaline, la duloxetine ou les inhibiteurs des canaux tels que les inhibiteurs de Nav 1,7.

15. Composé de formule (II) suivante :
**H-CO-N(OR)-CH₂-CH(R₁)-CO-NH-(CH₂)ₙ-CH(R₂)-(CH₂)ₘ-CO-R₃** **(II)**
dans lequel :
**R** représente :
- un groupement hydrocarboné linéaire ou ramifié comprenant de 1 à 6 atomes de carbone non substitué ou substitué par un ou plusieurs groupements aryles eux-mêmes non substitués ou substitués par un ou plusieurs groupements choisis parmi les aryles et les groupements alkyles linéaires ou ramifiés comprenant de 1 à 4 carbones, ou
- un groupement Si(R₁₀)₃, R₁₀ étant un groupement alkyle linéaire ou ramifié comprenant de 1 à 4 atomes de carbone,
**R₁** représente un groupement hydrocarboné linéaire ou ramifié comprenant de 1 à 6 atomes de carbone substitué par un ou plusieurs groupements choisis parmi :
- un aryle non substitué ou substitué par un ou plusieurs groupements choisis parmi les halogènes tels que le fluor et le brome, un groupement phényle, un groupement benzyle, un groupement OR₄, R₄ étant choisi parmi un hydrogène et un groupement alkyle linéaire ou ramifié comprenant de 1 à 4 atomes de carbone, et leurs combinaisons,
- un hétéroaryle à 5 ou 6 chaînons comprenant 1 ou 2 hétéroatomes, chaque hétéroatome étant choisi parmi l'oxygène, l'azote et le soufre, et
- un cyclohétéroalkyle à 5 ou 6 chaînons comprenant 1 ou 2 hétéroatomes, chaque hétéroatome étant choisi parmi l'oxygène, l'azote et le soufre,
**R₂** représente:
- un groupement hydrocarboné linéaire ou ramifié comprenant de 1 à 6 atomes de carbone, non substitué ou substitué par un ou plusieurs groupements choisis parmi :
• un groupement choisi parmi OR₅, SR₅ et S(O)R₅, R₅ étant choisi parmi un hydrogène et un groupement alkyle linéaire ou ramifié comprenant de 1 à 4 carbones,
• un groupement CO₂R₆, R₆ étant choisi parmi un hydrogène, un groupement alkyle linéaire ou ramifié comprenant de 2 à 4 atomes de carbone et un groupement benzyle,
• un hétéroaryle à 5 ou 6 chaînons comprenant 1 ou 2 hétéroatomes, chaque hétéroatome étant choisi parmi l'oxygène, l'azote et le soufre,
• un cycloalkyle à 5 ou 6 chaînons, et
• un cyclohétéroalkyle à 5 ou 6 chaînons comprenant 1 ou 2 hétéroatomes, chaque hétéroatome étant choisi parmi l'oxygène, l'azote et le soufre, ou
- un aryle, non substitué ou substitué par un ou plusieurs groupements choisis parmi les halogènes, tels que le fluor et le brome, un groupement OR₅, R₅ ayant la même définition que ci-dessus, et leurs combinaisons, ou
- un hétéroaryle comprenant 1 ou plusieurs hétéroatomes, de préférence 1 ou 2, chaque hétéroatome étant choisi parmi l'oxygène, l'azote et le soufre, non substitué ou substitué par un ou plusieurs groupements choisis parmi les halogènes, tels que le fluor et le brome, un groupement OR₅, R₅ ayant la même définition que ci-dessus, et leurs combinaisons,
**R₃** représente :
- un groupement OR₇, R₇ étant choisi parmi :
• un hydrogène,
• un groupement alkyle linéaire ou ramifié comprenant de 2 à 6 atomes de carbone
• un groupement benzyle, et
• un groupement CHR₈-COOR₉, CHR₈-O-C(=O)R₉ ou CHR₈₋O-C(=O)-OR₉ dans lesquels R₈ et R₉ sont, indépendamment l'un de l'autre, choisis parmi un groupement alkyle, un groupement aryle, un groupement arylalkyle, un groupement cycloalkyle, un groupement cyclohétéroalkyle, un groupement hétéroalkyle, un groupement hétéroaryle et un groupement hétéroarylalkyle, et
m et n sont indépendamment l'un de l'autre un nombre entier choisis parmi 0 et 1,
l'atome de carbone portant R**₁** ayant une configuration absolue (R) et l'atome de carbone portant **R₂** ayant une configuration absolue (S),
ainsi que leurs sels et/ou solvates pharmaceutiquement acceptables.

## Patentansprüche

1. Verbindung der folgenden Formel (I):
**H-CO-N (OH)-CH₂-CH(R₁)-CO-NH- (CH₂)ₙ-CH(R₂)- (CH₂)ₘ-CO- R₃** **(I)**
, in der:
**R₁** für eine gerade oder verzweigte Kohlenwasserstoffgruppe steht, umfassend 1 bis 6 Kohlenstoffatome, substituiert durch eine oder mehrere Gruppen, ausgewählt aus:
- einem Aryl, selbst unsubstituiert oder substituiert durch eine oder mehrere Gruppen, die aus den Halogenen wie Fluor und Brom, einer Phenylgruppe, einer Benzylgruppe, einer OR₄-Gruppe, wobei R₄ aus einem Wasserstoff und einer geraden oder verzweigten Alkylgruppe, umfassend 1 bis 4 Kohlenstoffatome ausgewählt ist, und deren Kombinationen ausgewählt sind,
- einem Heteroaryl mit 5 oder 6 Kettengliedern, umfassend 1 oder 2 Heteroatome, wobei jedes Heteroatom aus Sauerstoff, Stickstoff und Schwefel ausgewählt ist, und
- einem Cycloheteroalkyl mit 5 oder 6 Kettengliedern, umfassend 1 oder 2 Heteroatome, wobei jedes Heteroatom aus Sauerstoff, Stickstoff und Schwefel ausgewählt ist,
**R₂** steht für:
- eine gerade oder verzweigte Kohlenwasserstoffgruppe, umfassend 1 bis 6 Kohlenstoffatome, unsubstituiert oder substituiert durch eine oder mehrere Gruppen, ausgewählt aus:
• einer Gruppe, ausgewählt aus OR₅, SR₅ und S(O)R₅, wobei R₅ aus einem Wasserstoff und einer geraden oder verzweigten Alkylgruppe, umfassend 1 bis 4 Kohlenstoffatome, ausgewählt ist,
• einer CO₂R₆-Gruppe, wobei R₆ aus einem Wasserstoff, einer geraden oder verzweigten Alkylgruppe, umfassend 2 bis 4 Kohlenstoffatome, und einer Benzylgruppe ausgewählt ist,
• einem Aryl, selbst unsubstituiert oder substituiert durch eine oder mehrere Gruppen, die aus den Halogenen wie Fluor und Brom, einer OR₅-Gruppe, wobei R₅ dieselbe Definition wie oben aufweist, und deren Kombinationen ausgewählt sind,
• einem Heteroaryl mit 5 oder 6 Kettengliedern, umfassend 1 oder 2 Heteroatome, wobei jedes Heteroatom aus Sauerstoff, Stickstoff und Schwefel ausgewählt ist,
• einem Cycloalkyl mit 5 oder 6 Kettengliedern, und
• einem Cycloheteroalkyl mit 5 oder 6 Kettengliedern, umfassend 1 oder 2 Heteroatome, wobei jedes Heteroatom aus Sauerstoff, Stickstoff und Schwefel ausgewählt ist,
- ein Aryl, unsubstituiert oder substituiert durch eine oder mehrere Gruppen, die aus den Halogenen wie Fluor und Brom, einer OR₅-Gruppe, wobei R₅ dieselbe Definition wie oben aufweist, und deren Kombinationen ausgewählt sind, oder
- ein Heteroaryl, umfassend 1 oder mehrere Heteroatome, vorzugsweise 1 oder 2, wobei jedes Heteroatom aus Sauerstoff, Stickstoff und Schwefel ausgewählt ist, unsubstituiert oder substituiert durch eine oder mehrere Gruppen, die aus den Halogenen wie Fluor und Brom, einer OR₅-Gruppe, wobei R₅ dieselbe Definition wie oben aufweist, und deren Kombinationen ausgewählt sind,
**R₃** steht für:
- eine OR₇-Gruppe, wobei R₇ ausgewählt ist aus:
• einem Wasserstoff,
• einer geraden oder verzweigten Alkylgruppe, umfassend 2 bis 6 Kohlenstoffatome, einer Benzylgruppe, und
• einer CHR₈-COOR₉-, CHR₈-O-C (=O) R₉- oder CHRₑ-O-C (=O) -OR₉-Gruppe, in denen R₈ und R₉ unabhängig voneinander aus einer Alkylgruppe, einer Arylgruppe, einer Arylalkylgruppe, einer Cycloalkylgruppe, einer Cycloheteroalkylgruppe, einer Heteroalkylgruppe, einer Heteroarylgruppe und einer Heteroarylalkylgruppe ausgewählt sind, und
m und n unabhängig voneinander eine ganze Zahl sind, die aus 0 und 1 ausgewählt ist,
wobei das Kohlenstoffatom, das **R₁** trägt, eine absolute (R)-Konfiguration aufweist und das Kohlenstoffatom, das **R₂** trägt, eine absolute (S)-Konfiguration aufweist,
sowie deren pharmazeutisch unbedenklichen Salze und/oder Solvate.

2. Verbindung nach Anspruch 1, in der **R₁** für eine gerade oder verzweigte Kohlenwasserstoffgruppe steht, umfassend 1 bis 6 Kohlenstoffatome, substituiert durch ein Aryl, selbst unsubstituiert oder substituiert durch eine oder mehrere Gruppen, die aus den Halogenen wie Fluor und Brom, einer Phenylgruppe, einer Benzylgruppe, einer OR₄-Gruppe, wobei R₄ aus einem Wasserstoff und einer geraden oder verzweigten Alkylgruppe, umfassend 1 bis 4 Kohlenstoffatome ausgewählt ist, und deren Kombinationen ausgewählt sind.

3. Verbindung nach Anspruch 1 oder 2, in der **R₂** für eine gerade oder verzweigte Kohlenwasserstoffgruppe steht, umfassend 1 bis 6 Kohlenstoffatome, unsubstituiert oder substituiert durch eine oder mehrere Gruppen, ausgewählt aus:
• einem Aryl, selbst unsubstituiert oder substituiert durch eine oder mehrere Gruppen, die aus den Halogenen wie Fluor und Brom, einer OR₅-Gruppe, wobei R₅ dieselbe Definition wie in Anspruch 1 aufweist, und deren Kombinationen ausgewählt sind,
• einem Cycloalkyl mit 5 oder 6 Kettengliedern, und
• einem Cycloheteroalkyl mit 5 oder 6 Kettengliedern, umfassend 1 oder 2 Heteroatome, wobei jedes Heteroatom aus Sauerstoff, Stickstoff und Schwefel ausgewählt ist.

4. Verbindung nach einem der Ansprüche 1 bis 3, in der **R₃** für eine OR₇-Gruppe steht, wobei R₇ ausgewählt ist aus:
• einem Wasserstoff, und
• einer geraden oder verzweigten Alkylgruppe, umfassend 2 bis 6 Kohlenstoffatome.

5. Verbindung nach einem der Ansprüche 1 bis 4, in der:
**R₁** für eine gerade oder verzweigte Kohlenwasserstoffgruppe steht, umfassend 1 bis 6 Kohlenstoffatome, substituiert durch ein Aryl, selbst unsubstituiert oder substituiert durch eine oder mehrere Gruppen, die aus Fluor, Brom, einer Phenylgruppe, einer Benzylgruppe, einer OR₄-Gruppe, wobei R₄ aus einem Wasserstoff und einer geraden oder verzweigten Alkylgruppe, umfassend 1 bis 4 Kohlenstoffatome ausgewählt ist, und deren Kombinationen ausgewählt sind,
**R₂** für eine gerade oder verzweigte Kohlenwasserstoffgruppe von 1 bis 6 Kohlenstoffatomen steht, unsubstituiert oder substituiert durch eine oder mehrere Gruppen, ausgewählt aus:
• einem Aryl, selbst unsubstituiert oder substituiert durch eine oder mehrere Gruppen, die aus Fluor, Brom, einer OR₅-Gruppe, wobei R₅ dieselbe Definition wie in Anspruch 1 aufweist, und deren Kombinationen ausgewählt sind,
• einem Heteroaryl mit 5 oder 6 Kettengliedern, umfassend 1 oder 2 Heteroatome, wobei jedes Heteroatom aus Sauerstoff, Stickstoff und Schwefel ausgewählt ist,
• einem Cycloalkyl mit 5 oder 6 Kettengliedern, und
• einem Cycloheteroalkyl mit 5 oder 6 Kettengliedern, umfassend 1 oder 2 Heteroatome, wobei jedes Heteroatom aus Sauerstoff, Stickstoff und Schwefel ausgewählt ist,
**R₃** für eine OR₇-Gruppe steht, wobei R₇ ausgewählt ist aus:
• einem Wasserstoff, und
• einer geraden oder verzweigten Alkylgruppe, umfassend 2 bis 6 Kohlenstoffatome, und
m und n unabhängig voneinander eine ganze Zahl sind, die aus 0 und 1 ausgewählt ist.

6. Verbindung nach einem der Ansprüche 1 bis 5, in der **R₁** für eine Kohlenwasserstoffgruppe, vorzugsweise eine gerade oder verzweigte Alkylgruppe, umfassend 1 bis 6 Kohlenstoffatome, substituiert durch ein Aryl, vorzugsweise eine Phenylgruppe, selbst unsubstituiert oder substituiert durch eine Phenylgruppe, steht.

7. Verbindung nach einem der Ansprüche 1 bis 6, in der **R₂** für eine Kohlenwasserstoffgruppe, vorzugsweise eine gerade oder verzweigte Alkylgruppe, umfassend 1 bis 6 Kohlenstoffatome, unsubstituiert oder substituiert durch ein Aryl, steht.

8. Verbindung nach einem der Ansprüche 1 bis 7, in der **R₃** für eine OH-Gruppe steht.

9. Verbindung nach einem der Ansprüche 1 bis 8, in der:
- **R₁** = (R)-CH₂Ph; **R₂** = (S)-CH₃; **R₃** = OH; n = m = 0;
- **R₁** = (R)-CH₂Ph; **R₂** = (S)-CH₂Ph; **R₃** = OH; n = m = 0;
- **R₁** = (R)-CH₂Ph; **R₂** = (S)-CH₂Ph; **R₃** = OH; n = 0; m = 1;
- **R₁** = (R)-CH₂Ph-4-Ph; **R₂** = (S)-CH₃; **R₃** = OH; n = m = 0;
- **R₁** = (R)-CH₂Ph; **R₂** = (S)-CH₂Ph; **R₃** = OH; n = 1; m = 0.

10. Verbindung nach einem der Ansprüche 1 bis 9 zu deren Verwendung als ein Arzneimittel.

11. Verbindung nach einem der Ansprüche 1 bis 9 zu deren Verwendung als ein Analgetikum, Anxiolytikum oder Antidepressivum, insbesondere zur Behandlung von akuten oder chronischen Schmerzen, wie postoperativen, Krebs-, Unfall-, Kopfschmerzen, Migränen, viszeralen Schmerzen, neurogenen Schmerzen, Neuropathien, neuroinflammatorischen, nozizeptiven oder allgemeinen Schmerzen wie Fibromyalgie.

12. Pharmazeutische Zusammensetzung, umfassend mindestens eine Verbindung wie in einem der Ansprüche 1 bis 9 definiert und mindestens einen pharmazeutisch unbedenklichen Hilfsstoff.

13. Pharmazeutische Zusammensetzung nach Anspruch 12 zu deren Verwendung als ein Analgetikum, Anxiolytikum oder Antidepressivum, insbesondere zur Behandlung von akuten oder chronischen Schmerzen, wie postoperativen, Krebs-, Unfallschmerzen, viszeralen Schmerzen, Kopfschmerzen, Migränen, viszeralen Schmerzen, neurogenen Schmerzen, Neuropathien, neuroinflammatorischen, nozizeptiven oder allgemeinen Schmerzen wie Fibromyalgie.

14. Pharmazeutische Zusammensetzung nach Anspruch 12 oder pharmazeutische Zusammensetzung zu deren Verwendung nach Anspruch 13, weiterhin umfassend mindestens einen anderen Wirkstoff, insbesondere ein Analgetikum, das aus Morphin und seinen Derivaten, Endocannabinoiden und den Inhibitoren ihres Metabolismus, den Derivaten von GABA wie Gabapentin oder Pregabalin, Duloxetin oder Kanalinhibitoren wie den Inhibitoren von Nav 1.7.

15. Verbindung der folgenden Formel (II):
**H-CO-N(OR -CH₂-CH(R₁)-CO-NH-(CH₂)ₙ-CH(R₂) -(CH₂)ₘ-CO- R₃** **(II)**
, in der:
**R** steht für:
- eine gerade oder verzweigte Kohlenwasserstoffgruppe, umfassend 1 bis 6 Kohlenstoffatome, unsubstituiert oder substituiert durch eine oder mehrere Arylgruppen, selbst unsubstituiert oder substituiert durch eine oder mehrere Gruppen, die aus Arylen und geraden oder verzweigten Alkylgruppen, umfassend 1 bis 4 Kohlenstoffen, ausgewählt sind, oder
- eine Si (R₁₀) ₃-Gruppe, wobei R₁₀ eine gerade oder verzweigte Alkylgruppe, umfassend 1 bis 4 Kohlenstoffatome, ist,
**R₁** für eine gerade oder verzweigte Kohlenwasserstoffgruppe steht, umfassend 1 bis 6 Kohlenstoffatome, substituiert durch eine oder mehrere Gruppen, ausgewählt aus:
- einem Aryl, unsubstituiert oder substituiert durch eine oder mehrere Gruppen, die aus den Halogenen wie Fluor und Brom, einer Phenylgruppe, einer Benzylgruppe, einer OR₄-Gruppe, wobei R₄ aus einem Wasserstoff und einer geraden oder verzweigten Alkylgruppe, umfassend 1 bis 4 Kohlenstoffatome ausgewählt ist, und deren Kombinationen ausgewählt sind,
- einem Heteroaryl mit 5 oder 6 Kettengliedern, umfassend 1 oder 2 Heteroatome, wobei jedes Heteroatom aus Sauerstoff, Stickstoff und Schwefel ausgewählt ist, und
- einem Cycloheteroalkyl mit 5 oder 6 Kettengliedern, umfassend 1 oder 2 Heteroatome, wobei jedes Heteroatom aus Sauerstoff, Stickstoff und Schwefel ausgewählt ist,
**R₂** steht für:
- eine gerade oder verzweigte Kohlenwasserstoffgruppe, umfassend 1 bis 6 Kohlenstoffatome, unsubstituiert oder substituiert durch eine oder mehrere Gruppen, ausgewählt aus:
• einer Gruppe, ausgewählt aus OR₅, SR₅ und S(O)R₅, wobei R₅ aus einem Wasserstoff und einer geraden oder verzweigten Alkylgruppe, umfassend 1 bis 4 Kohlenstoffe, ausgewählt ist,
• einer CO₂R₆-Gruppe, wobei R₆ aus einem Wasserstoff, einer geraden oder verzweigten Alkylgruppe, umfassend 2 bis 4 Kohlenstoffatome, und einer Benzylgruppe ausgewählt ist,
• einem Heteroaryl mit 5 oder 6 Kettengliedern, umfassend 1 oder 2 Heteroatome, wobei jedes Heteroatom aus Sauerstoff, Stickstoff und Schwefel ausgewählt ist,
• einem Cycloalkyl mit 5 oder 6 Kettengliedern, und
• einem Cycloheteroalkyl mit 5 oder 6 Kettengliedern, umfassend 1 oder 2 Heteroatome, wobei jedes Heteroatom aus Sauerstoff, Stickstoff und Schwefel ausgewählt ist, oder
- ein Aryl, unsubstituiert oder substituiert durch eine oder mehrere Gruppen, die aus den Halogenen wie Fluor und Brom, einer OR₅-Gruppe, wobei R₅ dieselbe Definition wie oben aufweist, und deren Kombinationen ausgewählt sind, oder
- ein Heteroaryl, umfassend 1 oder mehrere Heteroatome, vorzugsweise 1 oder 2, wobei jedes Heteroatom aus Sauerstoff, Stickstoff und Schwefel ausgewählt ist, unsubstituiert oder substituiert durch eine oder mehrere Gruppen, die aus den Halogenen wie Fluor und Brom, einer OR₅-Gruppe, wobei R₅ dieselbe Definition wie oben aufweist, und deren Kombinationen ausgewählt sind,
**R₃** steht für:
- eine OR₇-Gruppe, wobei R₇ ausgewählt ist aus:
• einem Wasserstoff,
• einer geraden oder verzweigten Alkylgruppe, umfassend 2 bis 6 Kohlenstoffatome,
• einer Benzylgruppe, und
• einer CHR₈-COOR₉-, CHR₈-O-C(=O) R₉- oder CHR₈-O-C (=O) -OR₉-Gruppe, in denen R₈ und R₉ unabhängig voneinander aus einer Alkylgruppe, einer Arylgruppe, einer Arylalkylgruppe, einer Cycloalkylgruppe, einer Cycloheteroalkylgruppe, einer Heteroalkylgruppe, einer Heteroarylgruppe und einer Heteroarylalkylgruppe ausgewählt sind, und
m und n unabhängig voneinander eine ganze Zahl sind, die aus 0 und 1 ausgewählt ist,
wobei das Kohlenstoffatom, das **R₁** trägt, eine absolute (R)-Konfiguration aufweist und das Kohlenstoffatom, das **R₂** trägt, eine absolute (S)-Konfiguration aufweist,
sowie deren pharmazeutisch unbedenklichen Salze und/oder Solvate.

## Claims

1. A compound of the following formula (I):
**H-CO-N(OH)-CH₂-CH(R₁)-CO-NH-(CH₂)n-CH(R₂) -(CH₂)ₘCO- R₃,** **(I)**
in which:
**R₁** represents a linear or branched hydrocarbon group comprising 1 to 6 carbon atoms substituted by one or more groups chosen from:
- an aryl, itself not substituted or substituted by one or more groups chosen from the halogens such as fluorine and bromine, a phenyl group, a benzyl group, an OR4 group, R4 being chosen from a hydrogen and a linear or branched alkyl group comprising 1 to 4 carbon atoms, and the combinations thereof,
- a heteroaryl with 5 or 6 members comprising 1 or 2 heteroatoms, each heteroatom being chosen from oxygen, nitrogen and sulfur, and
- a cycloheteroalkyl with 5 or 6 members comprising 1 or 2 heteroatoms, each heteroatom being chosen from oxygen, nitrogen and sulfur,
**R₂** represents:
- a linear or branched hydrocarbon group comprising 1 to 6 carbon atoms, not substituted or substituted by one or more groups chosen from:
• a group chosen from OR₅, SR₅ and S(O)R₅, R₅ being chosen from hydrogen and a linear or branched alkyl group comprising 1 to 4 carbon atoms,
• a CO₂R₆ group, R₆ being chosen from hydrogen, a linear or branched alkyl group comprising 2 to 4 carbon atoms and a benzyl group,
• an aryl, itself not substituted or substituted by one or more groups chosen from the halogens, such as fluorine and bromine, an OR₅ group, R₅ having the same definition as above, and the combinations thereof,
• a heteroaryl with 5 or 6 members comprising 1 or 2 heteroatoms, each heteroatom being chosen from oxygen, nitrogen and sulfur,
• a cycloalkyl with 5 or 6 members, and
• a cycloheteroalkyl with 5 or 6 members comprising 1 or 2 heteroatoms, each heteroatom being chosen from oxygen, nitrogen and sulfur,
- an aryl, not substituted or substituted by one or more groups chosen from the halogens, such as fluorine and bromine, an OR₅ group, R₅ having the same definition as above, and the combinations thereof, or
- a heteroaryl comprising one or more, preferably 1 or 2, heteroatoms, each heteroatom being chosen from oxygen, nitrogen and sulfur, not substituted or substituted by one or more groups chosen from the halogens, such as fluorine and bromine, an OR₅ group, R₅ having the same definition as above, and the combinations thereof,
R₃, represents:
- an OR₇ group, R₇ being chosen from:
• hydrogen,
• a linear or branched alkyl group comprising 2 to 6 carbon atoms, a benzyl group, and
• a CHR₈-COOR₉, CHR8-O-C(=O)R₉ or CHR₈-O-C(=O)-OR₉ group, in which R₈ and R₉ are chosen, independently of one another, from an alkyl group, an aryl group, an arylalkyl group, a cycloalkyl group, a group cycloheteroalkyl, a heteroalkyl group, a heteroaryl group and a heteroarylalkyl group, and
m and n are, independently of one another, an integer chosen from 0 and 1, the carbon atom bearing **R₁** having an absolute configuration (R) and the carbon atom bearing **R₂** having an absolute configuration (S),
as well as their pharmaceutically acceptable salts and/or solvates.

2. The compound according to claim 1, in which **R₁** represents a linear or branched hydrocarbon group comprising 1 to 6 carbon atoms, substituted by an aryl, itself not substituted or substituted by one or more groups chosen from the halogens such as fluorine and bromine, a phenyl group, a benzyl group, an OR4 group, R4 being chosen from hydrogen and a linear or branched alkyl group comprising 1 to 4 carbon atoms, and the combinations thereof.

3. The compound according to claim 1 or 2, in which R₂ represents a linear or branched hydrocarbon group comprising 1 to 6 carbon atoms, not substituted or substituted by one or more groups chosen from:
• an aryl, itself not substituted or substituted by one or more groups chosen from the halogens such as fluorine and bromine, an OR₅ group, R₅ having the same definition as in claim 1, and the combinations thereof,
• a cycloalkyl with 5 or 6 members, and
• a cycloheteroalkyl with 5 or 6 members comprising 1 or 2 heteroatoms, each heteroatom being chosen from oxygen, nitrogen and sulfur.

4. The compound according to any one of claims 1 to 3, in which **R₃**, represents an OR₇ group, R₇ being chosen from:
• hydrogen, and
• a linear or branched alkyl group comprising 2 to 6 carbon atoms.

5. The compound according to any one of claims 1 to 4, in which:
**R₁** represents a linear or branched hydrocarbon group comprising 1 to 6 carbon atoms, substituted by an aryl, itself not substituted or substituted by one or more groups chosen from fluorine, bromine, a phenyl group, a benzyl group, an OR4 group, R4 being chosen from hydrogen and a linear or branched alkyl group comprising 1 to 4 carbon atoms, and the combinations thereof,
**R₂** represents a linear or branched hydrocarbon group of 1 to 6 carbon atoms, not substituted or substituted, by one or more groups chosen from:
• an aryl, itself not substituted or substituted by one or more groups chosen from fluorine, bromine, an OR₅ group, R₅ having the same definition as in claim 1, and the combinations thereof,
• a heteroaryl with 5 or 6 members comprising 1 or 2 heteroatoms, each heteroatom being chosen from oxygen, nitrogen and sulfur,
• a cycloalkyl with 5 or 6 members, and
• a cycloheteroalkyl with 5 or 6 members comprising 1 or 2 heteroatoms, each heteroatom being chosen from oxygen, nitrogen and sulfur,
**R₃,** represents an **OR₇** group, **R₇** being chosen from:
• hydrogen, and
• a linear or branched alkyl group comprising 2 to 6 carbon atoms, and m and n are, independently of one another, an integer chosen from 0 and 1.

6. The compound according to any one of claims 1 to 5, in which **R₁** represents a hydrocarbon group, preferably a linear or branched alkyl group comprising 1 to 6 carbon atoms, substituted by an aryl, preferably a phenyl group, itself not substituted or substituted by a phenyl group.

7. The compound according to any one of claims 1 to 6, in which **R₂** represents a hydrocarbon group, preferably a linear or branched alkyl group comprising 1 to 6 carbon atoms, not substituted or substituted by an aryl.

8. The compound according to any one of claims 1 to 7, in which **R₃** represents an OH group.

9. The compound according to any one of claims 1 to 8, in which:
- **R₁** = (R)-CH₂Ph; **R₂** = (S)-CH₃; **R₃** = OH; n=m=0;
- **R₁** = (R)-CH₂Ph; **R₂** = (S)-CH₂Ph; **R₃** = OH; n=m=0;
- **R₁** = (R)-CH₂Ph; **R₂** = (S)-CH₂Ph; **R₃** = OH; n=0; m=1;
- **R₁** = (R)-CH₂Ph-4-Ph; **R₂** = (S)-CH₃; **R₃** = OH; n=m=0;
- **R₁** = (R)-CH₂Ph; **R₂** = (S)-CH₂Ph; R₃ =OH; n=1; m=0.

10. The compound according to any one of claims 1 to 9, for the use thereof as a drug.

11. The compound according to any one of claims 1 to 9, for the use thereof as an analgesic, anxiolytic or antidepressant, in particular for the treatment of acute or chronic pain, such as post-operative, cancer-related or traumatic pain, headaches, migraines, visceral pain, neurogenic, neuropathic, neuro-inflammatory, nociceptive pain, or general pain such as fibromyalgia.

12. A pharmaceutical composition comprising at least one compound such as defined according to one of claims 1 to 9 and at least one pharmaceutically acceptable excipient.

13. The pharmaceutical composition according to claim 12 for the use thereof as an analgesic, anxiolytic or antidepressant, in particular for the treatment of acute or chronic pain, such as post-operative, cancer-related or traumatic pain, visceral pain, headaches, migraines, visceral pain, neurogenic, neuropathic, neuro-inflammatory, nociceptive pain, or general pain such as fibromyalgia.

14. The pharmaceutical composition according to claim 12 or pharmaceutical composition for the use thereof according to claim 13, further comprising at least one other active ingredient, in particular an analgesic chosen from morphine and its derivatives, endocannabinoids and the metabolism inhibitors thereof, derivatives of GABA such as gabapentin or pregabalin, duloxetine or channel blockers such as blockers of Nav 1.7.

15. A compound of the following formula (II):
**H-CO-N(OR)-CH₂-CH(R₁)-CO-NH-(CH₂)n-CH(R₂)-(CH₂)m- CO-R₃**, **(II)**
in which:
**R** represents:
- a linear or branched hydrocarbon group comprising 1 to 6 carbon atoms, not substituted or substituted by one or more aryl groups, themselves not substituted or substituted by one or more groups chosen from the aryls and linear or branched alkyl groups comprising 1 to 4 carbons, or
- an Si (R₁₀) 3 group, R₁₀ being a linear or branched alkyl group comprising 1 to 4 carbon atoms,
**R₁** represents a linear or branched hydrocarbon group comprising 1 to 6 carbon atoms substituted by one or more groups chosen from:
- an aryl, not substituted or substituted by one or more groups chosen from the halogens such as fluorine and bromine, a phenyl group, a benzyl group, an OR4 group, R4 being chosen from hydrogen and a linear or branched alkyl group comprising 1 to 4 carbon atoms, and the combinations thereof,
- a heteroaryl with 5 or 6 members comprising 1 or 2 heteroatoms, each heteroatom being chosen from oxygen, nitrogen and sulfur, and
- a cycloheteroalkyl with 5 or 6 members comprising 1 or 2 heteroatoms, each heteroatom being chosen from oxygen, nitrogen and sulfur,
**R₂** represents:
- a linear or branched hydrocarbon group comprising 1 to 6 carbon atoms, not substituted or substituted by one or more groups chosen from:
• a group chosen from OR₅, SR₅ and S(O)R₅, R₅ being chosen from hydrogen and a linear or branched alkyl group comprising 1 to 4 carbons,
• a CO₂R₆ group, R₆ being chosen from hydrogen, a linear or branched alkyl group comprising 2 to 4 carbon atoms and a benzyl group,
• a heteroaryl with 5 or 6 members comprising 1 or 2 heteroatoms, each heteroatom being chosen from oxygen, nitrogen and sulfur,
• a cycloalkyl with 5 or 6 members, and
• a cycloheteroalkyl with 5 or 6 members comprising 1 or 2 heteroatoms, each heteroatom being chosen from oxygen, nitrogen and sulfur, or
- an aryl, not substituted or substituted by one or more groups chosen from the halogens, such as fluorine and bromine, an OR₅ group, R₅ having the same definition as above, and the combinations thereof, or
- a heteroaryl comprising one or more, preferably 1 or 2, heteroatoms, each heteroatom being chosen from oxygen, nitrogen and sulfur, not substituted or substituted by one or more groups chosen from the halogens, such as fluorine and bromine, an OR₅ group, R₅ having the same definition as above, and the combinations thereof,
**R₃** represents:
- an OR₇ group, R₇ being chosen from:
• hydrogen,
• a linear or branched alkyl group comprising 2 to 6 carbon atoms
• a benzyl group, and
• a CHR₈-COOR₉, CHR₈-O-C(=O)R₉ or CHR₈-O-C(=O)-OR₉ group, in which R₈ and R₉ are chosen, independently of one another, from an alkyl group, an aryl group, an arylalkyl group, a cycloalkyl group, a group cycloheteroalkyl, a heteroalkyl group, a heteroaryl group and a heteroarylalkyl group, and
m and n are, independently of one another, an integer chosen from 0 and 1,
the carbon atom bearing **R₁** having an absolute configuration (R) and the carbon atom bearing **R₂** having an absolute configuration (S),
as well as their pharmaceutically acceptable salts and/or solvates.
